(19) Europäisches Patentamt
European Patent Office
Office européen des brevets

(11) Publication number: **0 376 959 B1**

(12) # EUROPEAN PATENT SPECIFICATION

(45) Date of publication of patent specification: **24.03.93**

(51) Int. Cl.5: **C07H 11/00**, C07H 23/00, C07F 17/02, C07F 15/00

(21) Application number: **88906550.4**

(22) Date of filing: **18.07.88**

(86) International application number: **PCT/US88/02353**

(87) International publication number: **WO 89/00574 (26.01.89 89/03)**

(54) **PLATINUM PHARMACEUTICALS.**

(30) Priority: **17.07.87 US 74825**
**14.01.88 US 143762**
**14.01.88 US 143761**

(43) Date of publication of application: **11.07.90 Bulletin 90/28**

(45) Publication of the grant of the patent: **24.03.93 Bulletin 93/12**

(84) Designated Contracting States: **AT BE CH DE FR GB IT LI LU NL SE**

(56) References cited: **US-A- 4 675 336**

(73) Proprietor: **GEORGETOWN UNIVERSITY**
**3800 Reservoir Road, Nothwest**
**Washington, DC 2007(US)**

(72) Inventor: **TALEBIAN, Abdolhossen**
**12136 Eddyspark Drive**
**Herndon, VA 22070(US)**
Inventor: **GREEN, Diana, C.**
**3101 South Manchester Road**
**Falls Church, VA 22044(US)**
Inventor: **HAMMER, Charles, F.**
**3524 Ouebec Street, Northwest**
**Washington, DC 20016(US)**
Inventor: **SCHEIN, Philip, S.**
**605 Old Gulph Road**
**Bryn Mawr, PA 19010(US)**

(74) Representative: **Le Guen, Gérard et al**
**CABINET LAVOIX 2, place d'Estienne d'Orves**
**F-75441 Paris Cédex 09 (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 376 959 B1

## Description

### BACKGROUND OF THE INVENTION

Platinum anti-cancer agents are known in the literature. One of the most well publicized of the platinum anti-cancer agents is cis-diammine-dichloroplatinum (II), also known as cis-DDP and cisplatin. A discussion of cisplatin and its usefulness in the treatment of various types of cancer, such as testicular carcinoma, bladder cancer, ovarian cancer, and head and neck cancer can be found in Zwelling, Cancer Chemotherapy, pp. 105-122 (1985).

Problems arise when such platinum agents are used in cancer treatment however. The toxicity of platinum to the bone marrow and kidneys precludes large sized dosages which can, in effect, render such treatment ineffective. Also, the overall desirability of and confidence in chemotherapy based upon known platinum active ingredients is decreased due to the drastic consequences to bone marrow and kidneys of the use of toxic levels of platinum.

### SUMMARY OF THE INVENTION

The present invention is directed toward platinum anti-cancer agents having increased water solubility. Such an increase in water solubility aids the body in passing the platinum out of the system, thus preserving healthy bone marrow and kidneys. The water solubility of the platinum anti-cancer agents is enhanced by the presence of a mono or disaccharide group on the platinum active ingredient compound.

Pharmaceutical compositions containing the active ingredient and methods of treating carcinoma by administering said compositions to patients suffering from carcinoma are also discussed.

### DETAILED DESCRIPTION OF THE INVENTION

In a first aspect of the present invention, there is provided a compound of the formula:

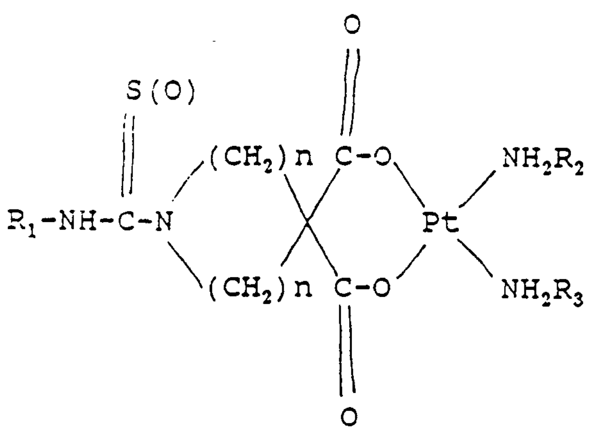

(I)

wherein n is 1 or 2; $R_1$ is a mono or disaccharide or derivative thereof; each of $R_2$ and $R_3$ is independently selected from the group consisting of hydrogen or $C_{1-4}$ alkyl, or $R_2$ and $R_3$ together are linked to adjacent carbon atoms on a five or six membered ring structure

or a pharmaceutically acceptable salt thereof.

The symbol "(O)" next to the sulfur atom indicates that an oxygen atom may replace the sulfur atom in the structure of the present invention.

As a mono or disaccharide of the present invention there is contemplated any conventional mono or disaccharide. The saccharides may be in pyranosyl or furanosyl form. Preferred form for the saccharides of the present invention is the pyranosyl form. Exemplary monosaccharides are glucose, mannose, galactose, sedoheptulose, sorbose, fructose, ribulose, and xylulose. Exemplary disaccharides are sucrose, lactose, cellobiose, maltose and isomaltose.

As said derivative of the mono or disaccharides there may be mentioned sugar alcohols, deoxy sugars, glyconic acids, glycuronic acids, glycosides, acetyl substituted, amino substituted, N-acetylamino substituted. Combinations of the various aforementioned substituents on one saccharide are also contemplated. For example, a 2-(N-acetylamino)-3,4,6-tri-O-acetyl-2-deoxyglucopyranosyl saccharide moiety is contemplated by the present invention.

As a five or six membered ring structure, there is contemplated a substituted or unsubstituted cyclohexyl or cyclopentyl ring system. The substituents thereon are such that they do not interfere with the anti-cancer activity of the compound. Exemplary of such substituents are $C_{1-4}$ alkyl, hydroxy.

Also contemplated are heterocyclic five or six membered rings having one or more of either nitrogen, oxygen or sulfur or a combination thereof. Exemplary of such rings are furan, pyran, piperidine.

As a pharmaceutically acceptable salt there is contemplated any salt that is safe for ingestion or injection and that is biologically inert, and hence does not interfere with the active ingredient. As such pharmaceutically acceptable salts may be mentioned sulfates, phosphates.

A preferred embodiment of the first aspect of the present invention involves a compound of the formula (I), wherein $R_1$ is a mono or disaccharide or derivative thereof selected from the group consisting of glucose, galactose, mannose, glucosamine and galactosamine and derivatives thereof.

Another preferred embodiment of the first aspect of the present invention involves a compound formula (I), wherein $R_2$ and $R_3$ are hydrogen.

Still another preferred embodiment of the first aspect of the present invention involves a compound of formula (I), wherein $R_2$ and $R_3$ together are linked to adjacent carbon atoms on a five or six membered ring structure.

Another preferred embodiment of the first aspect of the present invention involves a compound of the formula:

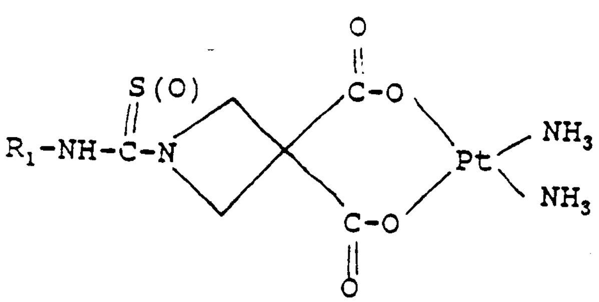

(II)

and $R_1$ is selected from the group comprising glucose, mannose, galactose, glucosamine, galactosamine and derivatives thereof.

Further preferred in a first aspect of the present invention is a compound, wherein the compound is of the formula:

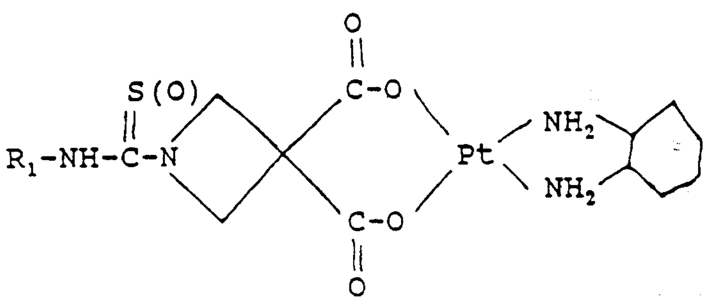

(III)

and $R_1$ is selected from the group comprising glucose, mannose, galactose, glucosamine, galactosamine and derivatives thereof.

Additionally preferred in the first aspect of the present invention is a compound, wherein the compound is of the formula:

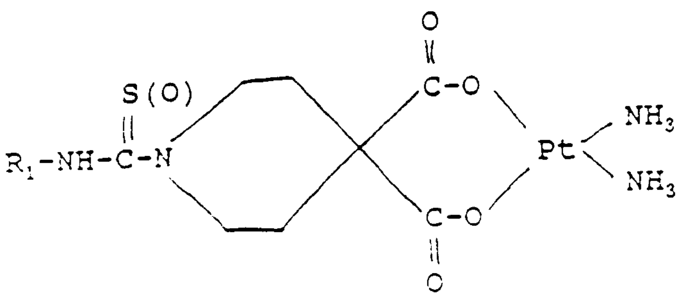

(IV)

and $R_1$ is selected from the group comprising glucose, mannose, galactose, glucosamine, galactosamine and derivatives thereof.

Another preferred embodiment of the first aspect of the invention is a compound, wherein the compound is of the formula:

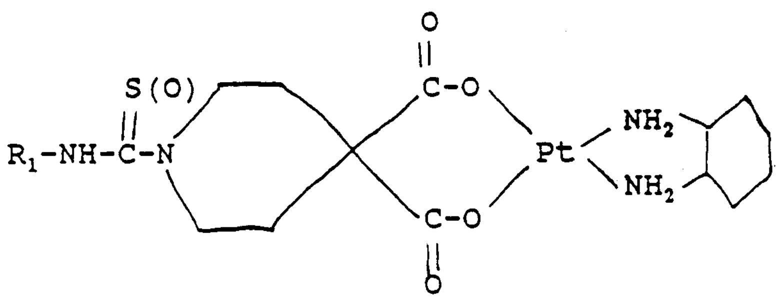

(V)

and $R_1$ is selected from the group comprising glucose, mannose, galactose, glucosamine, galactosamine and derivatives thereof.

A second aspect of the present invention involves a compound of the formula:

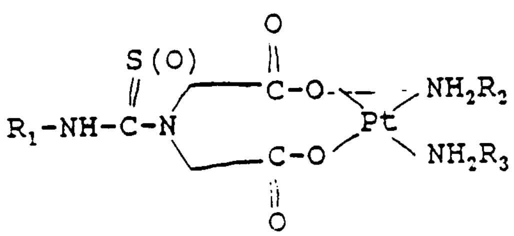

(VI)

wherein $R_1$ is a mono or disaccharide or a derivative thereof, $R_2$ and $R_3$ are selected from the group consisting of hydrogen, $C_{1-4}$ alkyl or $R_2$ and $R_3$ or $R_2$ and $R_3$ together are linked to adjacent carbon atoms on a five or six membered ring structure

or a pharmaceutically acceptable salt thereof.

As a mono or disaccharide of the present invention there is contemplated any conventional mono or disaccharide. The saccharides may be in pyranosyl or furanosyl form. Preferred form for the saccharides of the present invention is the pyranosyl form. Exemplary monosaccharides are glucose, mannose, galactose, sedoheptulose, sorbose, fructose, ribulose, and xylulose. Exemplary disaccharides are sucrose, lactose, cellobiose, maltose and isomaltose.

As said derivative of the mono or disaccharides there may be mentioned sugar alcohols, deoxy sugars, glyconic acids, glycuronic acids, glycosides, acetyl substituted, amino substituted, N-acetylamino substituted, and the like. Combinations of the various aforementioned substituents on one saccharide are also contemplated. For example, a 2-(N-acetylamino)-3,4,6-trio-O-acetyl-2-deoxyglucopyranosyl saccharide moiety is contemplated by the present invention.

As a five or six membered ring structure, there is contemplated a substituted or unsubstituted cyclohexyl or cyclopentyl ring system. The substituents thereon are such that they do not interfere with the anti-cancer activity of the compound. Exemplary of such substituents are $C_{1-4}$ alkyl, hydroxy.

Also contemplated are heterocyclic five or six membered rings having one or more of either nitrogen, oxygen or sulfur or a combination thereof. Exemplary of such rings are furan, pyran, piperidine.

As a pharmaceutically acceptable salt there is contemplated any salt that is safe for ingestion or injection and that is biologically inert, and hence does not interfere with the active ingredient. As such pharmaceutically acceptable salts may be mentioned sulfates, phosphates.

A preferred embodiment of the second aspect of the present invention involves a compound of the formula (VI), wherein $R_1$ is a mono or disaccharide or derivative thereof selected from the group consisting of glucose, galactose, mannose, glucosamine and galactosamine and derivatives thereof.

Another preferred embodiment of the second aspect of the present invention involves a compound of formula (VI), wherein $R_2$ and $R_3$ are hydrogen.

Still another preferred embodiment of the second aspect of the present invention involves a compound of formula (VI), wherein $R_2$ and $R_3$ together are linked to adjacent carbon atoms on a five or six membered ring structure.

Further preferred in the second embodiment is a compound, wherein the compound is of the formula:

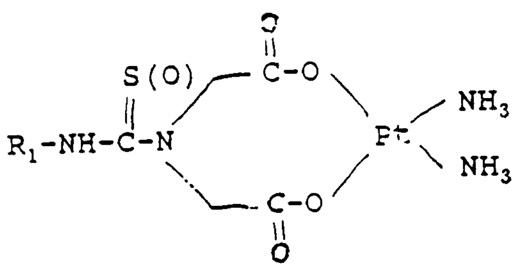

(VII)

and $R_1$ is selected from the group comprising glucose, mannose, galactose, glucosamine, galactosamine and derivatives thereof.

Additionally preferred in the second embodiment is a compound, wherein the compound is of the formula:

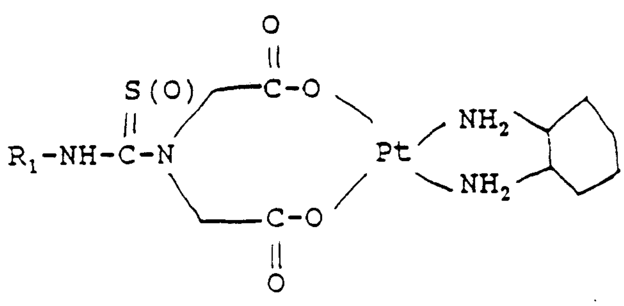

(VIII)

and $R_1$ is selected from the group comprising glucose, mannose, galactose, glucosamine, galactosamine and derivatives thereof.

Also preferred within the second aspect of the present invention is a compound of the formula:

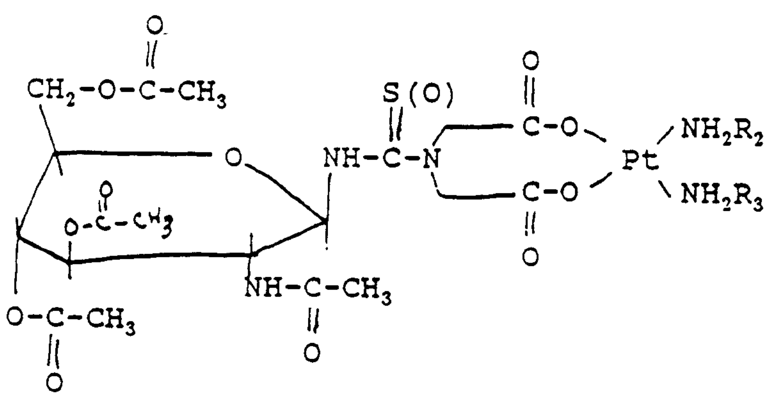

(IX)

wherein $R_2$ and $R_3$ are as defined above.

In a third aspect of the present invention, there is provided a compound of the formula:

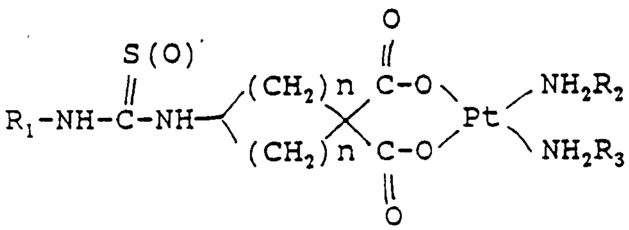

(X)

wherein n is 1 or 2; $R_1$ is a mono or disaccharide or derivative thereof; each of $R_2$ and $R_3$ is independently selected from the group consisting of hydrogen or $C_{1-4}$ alkyl, or $R_2$ and $R_3$ together are linked to adjacent carbon atoms on a five or six membered ring structure

or a pharmaceutically acceptable salt thereof.

As a mono or disaccharide of the present invention there is contemplated any conventional mono or disaccharide. The saccharides may be in pyranosyl or furanosyl form. Preferred form for the saccharides of the present invention is the pyranosyl form. Exemplary monosaccharides are glucose, mannose, galactose, sedoheptulose, sorbose, fructose, ribulose, and xylulose. Exemplary disaccharides are sucrose, lactose, cellobiose, maltose and isomaltose.

As said derivative of the mono or disaccharides there may be mentioned sugar alcohols, deoxy sugars, glyconic acids, glycuronic acids, glycosides, acetyl substituted, amino substituted, N-acetylamino substituted. Combinations of the various aforementioned substituents on one saccharide are also contemplated. For example, a 2-(N-acetylamino)-3,4,6-tri-O-acetyl-2-deoxyglucopyranosyl saccharide moiety is contemplated by the present invention.

As a five or six membered ring structure, there is contemplated a substituted or unsubstituted cyclohexyl or cyclopentyl ring system. The substituents thereon are such that they do not interfere with the anti-cancer activity of the compound. Exemplary of such substituents are $C_{1-4}$ alkyl, hydroxy.

Also contemplated are heterocyclic five or six membered rings having one or more of either nitrogen, oxygen or sulfur or a combination thereof. Exemplary of such rings are furan, pyran, piperidine.

As a pharmaceutically acceptable salt there is contemplated any salt that is safe for ingestion or injection and that is biologically inert, and hence does not interfere with the active ingredient. As such pharmaceutically acceptable salts may be mentioned sulfates, phosphates.

A preferred embodiment of this aspect involves a compound, wherein said compound is of the formula:

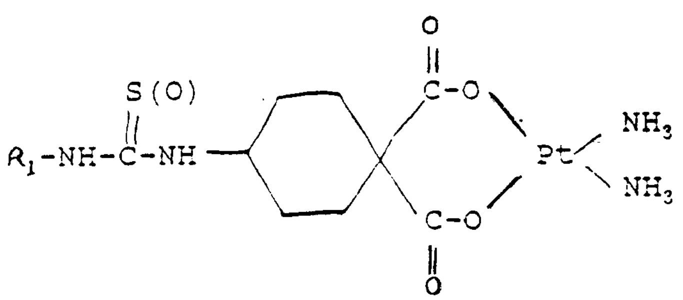

(XI)

and $R_1$ is selected from the group comprising glucose, mannose, galactose, glucosamine, galactosamine and derivatives thereof.

An additional preferred embodiment of the present invention involves a compound, wherein said compound is of the formula:

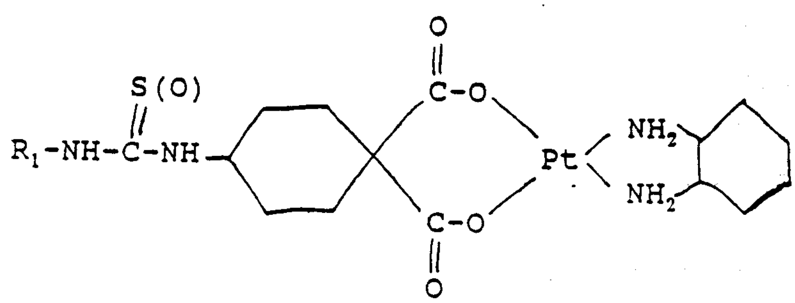

(XII)

and $R_1$ is selected from the group comprising glucose, mannose, galactose, glucosamine, galactosamine and derivatives thereof.

In a fourth aspect of the present invention, there is provided a compound of the formula

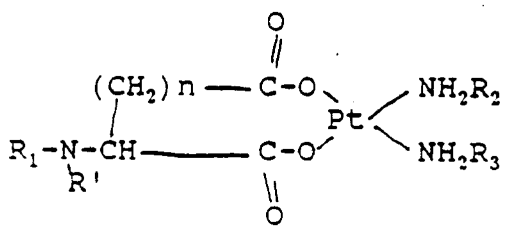

(XIII)

wherein n is 0 or 1, $R_1$ is selected from the group consisting of hydrogen, a mono or disaccharide or a derivative thereof linked to the nitrogen atom by a -NHCO- amide moiety, an -NHCS- thioamide moiety, or a -CO- carbonyl moiety, R' is selected from the group consisting of hydrogen and $C_{1-4}$ alkyl or $R_2$ and $R_3$ or $R_2$ and $R_3$ together are linked to adjacent carbon atoms on a four, five or six membered ring structure, or $R_2$ and $R_3$ together form a fused or bicyclic ring with adjacent carbon atoms; with the proviso that R' and $R_1$ cannot both be hydrogen when $n = 0$, or a pharmaceutically acceptable salt thereof.

As a mono or disaccharide of the present invention there is contemplated any conventional mono or disaccharide. The saccharides may be in pyranosyl or furanosyl form. Preferred form for the saccharides of the present invention is the pyranosyl form. Exemplary monosaccharides are glucose, mannose, galactose, sedoheptulose, sorbose, fructose, ribulose, and xylulose. Exemplary disaccharides are sucrose, lactose, cellobiose, maltose and isomaltose. As said derivative of the mono or disaccharides there may be mentioned sugar alcohols, deoxy sugars, glyconic acids, glycuronic acids, glycosides, acetyl substituted, amino substituted, N-acetylamino substituted. Combinations of the various aforementioned substituents on one saccharide are also contemplated. For example, a 2-(N-acetylamino)-3,4,6-tri-O-acetyl-2-deoxyglucopyranosyl saccharide moiety is contemplated by the present invention.

As a five or six membered ring structure, there is contemplated a substitute or unsubstituted cyclohexyl or cyclopentyl ring system. The substituents thereon are such that they do not interfere with the anti-cancer activity of the compound. Exemplary of such substitutents are $C_{1-4}$ alkyl, hydroxy.

Also contemplated are heterocyclic five or six membered rings having one or more of either nitrogen, oxygen or sulfur or a combination thereof. Exemplary of such rings are furan, pyran, piperidine.

As a fused or polycyclic ring there are contemplated rings of the following formulae:

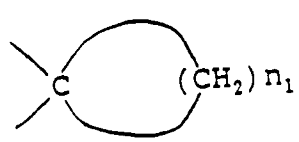

and

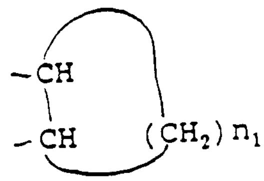

wherein $n_1$ is selected from 1, 2, 3, 4, 5, or 6.

As a pharmaceutically acceptable salt there is contemplated any salt that is safe for ingestion or injection and that is biologically inert, and hence does not interfere with the active ingredient. As such pharmaceutically acceptable salts may be mentioned sulfates, phosphates.

A preferred embodiment of the third aspect of the present invention involves a compound of the formula (X), wherein $R_1$ is a mono or disaccharide or derivative thereof selected from the group consisting of glucose, galactose, mannose, glucosamine and galactosamine and derivatives thereof.

Another preferred embodiment of this aspect of the present invention involves a compound of formula (X), wherein $R_2$ and $R_3$ are hydrogen.

Still another preferred embodiment of the fourth aspect of the present invention involves a compound of formula (X), wherein $R_2$ and $R_3$ together are linked to adjacent carbon atoms on a five or six membered ring structure.

Further preferred in this aspect is a compound, wherein the compound is of the formula:

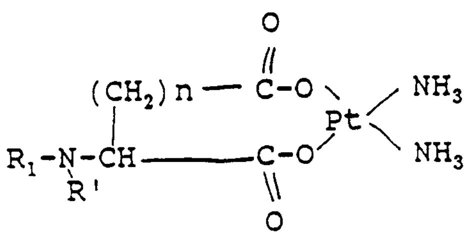

(XIV)

Also embodied in this aspect of the present invention is a compound, wherein the compound is of the formula:

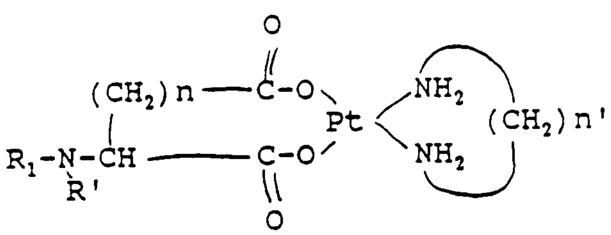

(XV)

wherein n' is 1, 2 or 3.

Another embodiment of this aspect of the present invention contemplates a compound, wherein $R_2$ and $R_3$ together form a fused or bicyclic ring with adjacent carbon atoms.

A preferred embodiment of the present invention involves a compound, wherein said compound is (L-aspartato-0-0')-(1,2-cyclohexanediammine-N,N')-platinum (II).

An additional embodiment involves a compound, wherein said compound is diammine-2-[[[[3,4,6-tri-0-acetyl-2-(N-acetylamino)-2-deoxy-alpha-D-glucopyranosyl]amino] thioxomethyl]amino]butanedioato-0-0']-platinum (II).

A further embodiment of the present invention involves a compound, wherein said compound is 2-[[[-[3,4,6-tri-0-acetyl-2-(N-acetylamino)-2-deoxy-alpha-D-glucopyranosyl] amino]thioxmethyl]amino]-butanedioato-0-0']-(1,2-cyclohexanediammine-N,N'-platinum (II).

In accordance with the present invention a pharmaceutical composition for the treatment of ailments consisting of testicular cancer, cancer of the ovary, head and neck cancer, cancer of the bladder and cancer

of the colon comprising a pharmaceutically effective amount of a compound of the formula (I) and a pharmaceutically acceptable carrier therefor.

The active ingredient is admixed with a pharmaceutically acceptable solid or liquid carrier to allow oral, parenteral, intramuscular or intravenous administration of effective amounts of the pharmaceutical.

As a dosage form for oral delivery there is contemplated any dosage form capable of being delivered orally. That is, tablets, coated tablets, capsules, caplets or any other oral dosage form are contemplated by the present invention.

As said pharmaceutically acceptable inert ingredients there are contemplated pharmaceuticals, carriers, excipients, fillers, etc. which do not interfere with the anti-cancer activity of said compound.

Fillers such as clays or siliceous earth may be utilized if desired to adjust the size of the dosage form. Further ingredients such as excipients and carriers may be necessary to impart the desired physical properties of the dosage form. Such physical properties are, for example, release rate, texture and size of the dosage form. Examples of excipients and carriers useful in oral dosage forms are waxes such as beeswax, castor wax glycowax and carnauba wax, cellulose compounds such as methylcellulose, ethylcellulose, carboxymethylcellulose, cellulose acetate phthalate, hydroxypropylcellulose and hydroxypropylmethylcellulose, polyvinyl chloride, polyvinyl pyrrolidone, stearyl alcohol, glycerin monostearate, methacrylate compounds such as polymethacrylate, methyl methacrylate and ethylene glycol dimethacrylate, polyethylene glycol and hydrophilic gums.

As an intraperitoneal, intramuscular or intravenous dosage form there is contemplated any dosage form safe for injection purposes and capable of delivering the active platinum containing compound to a patient suffering from ailments consisting of testicular cancer, cancer of the ovary, head and neck cancer, cancer of the bladder and cancer of the colon. Exemplary of such a solution is an isotonic solution. An isotonic solution of the invention may contain in addition to said compound, water and salt, also conventional ingredients such as glucose.

A preferred composition of the present invention involves a composition, wherein said compound, i.e. active ingredient, is of formula (I) and is such that $R_1$ is a mono or disaccharide or derivative thereof selected from the group consisting of glucose, galactose, mannose, glucosamine and galactosamine and derivatives thereof.

Another preferred composition of the present invention involves a composition, wherein said compound is of formula (I), and is such that $R_2$ and $R_3$ together are linked to adjacent carbon atoms on a five or six membered ring structure.

Additional preferred compositions of the present invention involve compositions, wherein the active compound therein is a compound of formulae (II), (III), (IV) and (V).

Also, in accordance with the present invention a pharmaceutical composition for the treatment of ailments consisting of testicular cancer, cancer of the ovary, head and neck cancer, cancer of the bladder and cancer of the colon comprising a pharmaceutically effective amount of a compound of the formula (VI) and a pharmaceutically acceptable carrier therefor.

The active Ingredient is admixed with a pharmaceutically acceptable solid or liquid carrier to allow oral, parenteral, intramuscular or intravenous administration of effective amounts of the pharmaceutical. The oral, parenteral, intramuscular and intravenous dosage forms may be those discussed above.

A preferred composition of the present invention involves a composition, wherein said compound, i.e.active ingredient, and is of formula (VI) is such that $R_1$ is a mono or disaccharide or derivative thereof selected from the group consisting of glucose, galactose, mannose, glucosamine and galactosamine and derivatives thereof.

Another preferred composition of the present invention involves a composition, wherein said compound is of formula (VI) and is such that R2 and $R_3$ are hydrogen.

Still another preferred composition the present invention involves a composition, wherein said compound is of formula (VI) and is such that $R_2$ and $R_3$ together are linked to adjacent carbon atoms on a five or six membered ring structure.

Additional preferred compositions of the present invention involve compositions, wherein the active compound therein is a compound of formulae (VII), (VIII) and (IX).

Also, in accordance with the present invention a pharmaceutical composition for the treatment of ailments consisting of testicular cancer, cancer of the ovary, head and neck cancer, cancer of the bladder and cancer of the colon comprising a pharmaceutically effective amount of a compound of the formula (X) and a pharmaceutically acceptable carrier therefor.

The active ingredient is admixed with a pharmaceutically acceptable solid or liquid carrier to allow oral, parenteral, intramuscular or intravenous administration of effective amounts of the pharmaceutical. The oral, parenteral, intramuscular and intravenous dosage forms may be those discussed above.

Preferred compositions of the present invention involve compositions, wherein the active compound therein is a compound of formulae (XI) and (XII).

Moreover, in accordance with the present invention a pharmaceutical composition for the treatment of ailments consisting of testicular cancer, cancer of the ovary, head and neck cancer, cancer of the bladder and cancer of the colon comprising pharmaceutically effective amount of a compound of the formula (XIII) and a pharmaceutically acceptable carrier therefor.

The active ingredient is admixed with a pharmaceutically acceptable solid or liquid carrier to allow oral, parenteral, intramuscular or intravenous administration of effective amounts of the pharmaceutical. The oral, parenteral, intramuscular and intravenous dosage forms may be those discussed above.

A preferred composition of the present invention involves a composition, wherein said compound, i.e. active ingredient, and is of formula (XIII) is such that $R_1$ is a mono or disaccharide or derivative thereof selected from the group consisting of glucose, galactose, mannose, glucosamine and galactosamine and derivatives thereof.

Another preferred composition of the present invention involves a composition, wherein said compound is of formula (XIII) and is such that $R_2$ and $R_3$ are hydrogen.

Still another preferred composition the present invention involves a composition, wherein said compound is of formula (XIII) and is such that $R_2$ and $R_3$ together are linked to adjacent carbon atoms on a five or six membered ring structure.

Additional preferred compositions of the present invention involve compositions, wherein the active compound therein is a compound of formulae (XIV) and (XV), (L-aspartato-0-0')-(1,2-cyclohexanediammine)-N,N'-platinum (II), diammine-2-[[[[3,4,6-tri-0-acetyl-2-(N-acetylamino)-2-deoxy-alpha-D-glucopyranosyl]-amino]thioxomethyl]amino] butanedioato-0,0']-platinum (II), and 2-[[[[3,4,6-tri-0-acetyl-2-(n-acetylamino)-2-deoxy-alpha-D-glucopyranosyl] amino]thioxomethyl]amino]butanedioato-0-0']-(1,2-cyclohexanediammine-N,N')-platinum (II).

Further in accordance with the present invention there is provided a method for the treatment of ailments consisting of testicular cancer, cancer of the ovary, head and neck cancer, cancer of the bladder and cancer of the colon comprising administration of a pharmaceutically effective amount of a compound of the formula (I) and a pharmaceutically acceptable carrier therefor to a patient suffering from said ailments.

The administration can occur through oral, intraperitoneal, intramuscular and intravenous routes. Therapeutic treatment profiles can be arranged to administer the compound in accordance with the need of the patient. The need of the patient is dependent on typical factors such as the advancement of the disease, the patient's age, general health, and the like. Daily, weekly, or dosing every two or three weeks are exemplary of possible treatment protocols. With respect to intravenous administration, the compound could be administered constantly. Periods up to 7 days are exemplary of possible intravenous treatment protocols.

Regardless of mode of administration, an exemplary dose of the active compound is from about 1 to about 1000 mg per $m^2$ body surface area of a patient. A preferred dosage of the active compound involves the administration of about 10 to about 200 mg per $m^2$ body surface area of a patient. A more preferred dosage of the active compound involves the administration or about 50 to about 150 mg per $m^2$ body surface area of a patient.

A preferred method of the present invention involves the administration of a compound of formula (I), wherein said compound is such that $R_1$ is a mono or disaccharide or derivative thereof selected from the group consisting of glucose, galactose, mannose, glucosamine and galactosamine and derivatives thereof.

Another preferred method of the present invention involves the administration of a compound of formula (I), wherein said compound is such that $R_2$ and $R_3$ are hydrogen.

A further method of the present invention involves the administration of a compound of formula (I), wherein said compound is such that $R_2$ and $R_3$ together are linked to adjacent carbon atoms on a five or six membered ring structure.

Additional preferred methods of the present invention involve the administration of a compound, wherein the compound therein is a compound of formulae (II), (III), (IV), and (V).

Further in accordance with the present invention there is provided a method for the treatment of ailments consisting of testicular cancer, cancer of the ovary, head and neck cancer, cancer of the bladder and cancer of the colon comprising administration of a pharmaceutically effective amount of a compound of the formula (VI) and a pharmaceutically acceptable carrier therefor to a patient suffering from said ailment.

The administration can occur through oral, intraperitoneal, intramuscular and intravenous routes. Therapeutic treatment profiles can be arranged to administer the compound in accordance with the need of the patient. The need of the patient is dependent on typical factors such as the advancement of the disease, the patient's age, general health, and the like. Daily, weekly, or dosing every two or three weeks

are exemplary of possible treatment protocols. With respect to intravenous administration, the compound could be administered constantly. Periods up to 7 days are exemplary of possible intravenous treatment protocols.

Regardless of mode of administration, an exemplary dose of the active compound is from about 1 to about 1000 mg per $m^2$ body surface area of a patient. A preferred dosage of the active compound involves the administration of about 10 to about 200 mg per $m^2$ body surface area of a patient. A more preferred dosage of the active compound involves the administration of about 50 to about 150 mg per $m^2$ body surface area of a patient.

A preferred method of the present invention involves the administration of a compound of formula (VI), wherein said compound is such that $R_1$ is a mono or disaccharide or derivative thereof selected from the group consisting of glucose, galactose, mannose, glucosamine and galactosamine and derivatives thereof.

Another preferred method of the present invention involves the administration of a compound of formula (VI), wherein said compound is such that $R_2$ and $R_3$ are hydrogen.

A further method of the present invention involves the administration of a compound of formula (VI), wherein said compound is such that $R_2$ and $R_3$ together are linked to adjacent carbon atoms on a five or six membered ring structure.

Additional preferred methods of the present invention involve administration of compounds, wherein the compound therein is a compound of formulae (VII), (VIII) and (IX).

Further in accordance with the present invention there is provided a method for the treatment of ailments consisting of testicular cancer, cancer of the ovary, head and neck cancer, cancer or the bladder and cancer of the colon comprising administration of a pharmaceutically effective amount of a compound of the formula (X) and a pharmaceutically acceptable carrier therefor to a patient suffering from said ailment.

The administration can occur through oral, intraperitoneal, intramuscular and intravenous routes. Therapeutic treatment profiles can be arranged to administer the compound in accordance with the need of the patient. The need of the patient is dependent on typical factors such as the advancement of the disease, the patient's age, general health, and the like. Daily, weekly, or dosing every two or three weeks are exemplary of possible treatment protocols. With respect to intravenous administration, the compound could be administered constantly. Periods up to 7 days are exemplary of possible intravenous treatment protocols.

Regardless of mode of administration, an exemplary dose of the active compound is from about 1 to about 1000 mg per $m^2$ body surface area of a patient. A preferred dosage of the active compound involves the administration of about 10 to about 200 mg per $m^2$ body surface area of a patient. A more preferred dosage of the active compound involves the administration of about 50 to about 150 mg per $m^2$ body surface area of a patient.

Additional preferred methods of the present invention involve administration of compounds, wherein the compound therein is a compound of formulae (XI) and (XII).

Still further in accordance with the present invention there is provided a method for the treatment of ailments consisting of testicular cancer, cancer of the ovary, head and neck cancer, cancer of the bladder and cancer of the colon comprising administration of a pharmaceutically effective amount of a compound of the formula (XIII) and a pharmaceutically acceptable carrier therefor to a patient suffering from said ailments.

The administration can occur through oral, intraperiotoneal, intramuscular and intravenous routes. Therapeutic treatment profiles can be arranged to administer the compound in accordance with the need of the patient. The need of the patient is dependent on typical factors such as the advancement of the disease, the patient's age, general health, and the like. Daily, weekly, or dosing every two or three weeks are exemplary of possible treatment protocols. With respect to intravenous administration, the compound could be administered constantly. Periods up to 7 days are exemplary of possible intravenous treatment protocols.

Regardless of mode of administration, an exemplary dose of the active compound is from about 1 to about 1000 mg per $m^2$ body surface area of a patient. A preferred dosage of the active compound involves the administration of about 10 to about 200 mg per $m^2$ body surface area of a patient. A more preferred dosage of the active compound involves the administration of about 50 to about 150 mg per $m^2$ body surface area of a patient.

A preferred method of the present invention involves the administration of a compound of formula (XIII), wherein said compound is such that $R_1$ is a mono or disaccharide or derivative thereof selected from the group consisting of glucose, galactose, mannose, glucosamine and galactosamine and derivatives thereof.

Another preferred method of the present invention involves the administration of a compound of formula (XIII), wherein said compound is such that $R_2$ and $R_3$ are hydrogen.

A further method of the present invention involves the administration of a compound of formula (XIII), wherein said compound is such that $R_2$ and $R_3$ together are linked to adjacent carbon atoms on a five or six membered ring structure.

Additional preferred methods of the present invention involve the administration of a compound, wherein the compound therein is a compound of formulae (XIV) and (XV), (L-aspartato-0,0')-(1,2-cyclohexanediammine)-N,N'-platinum (II), diammine-2-[[[[3,4,6-tri-0-acetyl-2-(N-acetylamino)-2-deoxy-alpha-D-glucopyranosyl]amino]thioxomethyl]amino] butanedioato-0-0']-platinum (II), and 2-[[[[3,4,6-tri-0-acetyl-2-(N-acetylamino)-2-deoxy-alpha-D-glucopyranosyl] amino]thioxmethyl]amino]butanedioato-0,0']-(1,2-cyclohexanediammine-N,n')-platinum (II).

The thio compounds of formula (I) of the present invention may be prepared according to the following reaction scheme:

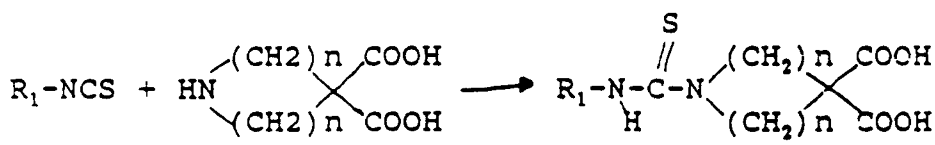

1 2 3

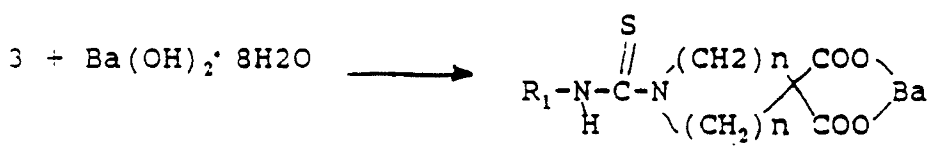


4

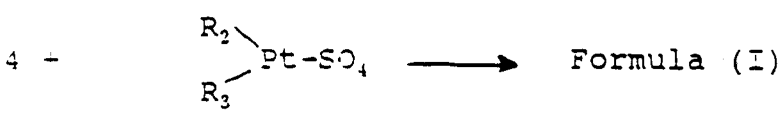


The oxo derivatives can be made in accordance with an analogous method with an $R_1$-NCO starting material.

The compound of formula (VI) may also be made in accordance with the above reactant 2 into the first reaction step.

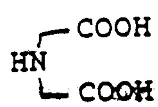

The compounds of the third aspect of the present invention can be prepared according to an analogous reaction mechanism utilizing a cycloalkyl containing starting material.

The thio compounds of formula (XIII) may be prepared in accordance with the following reaction scheme.

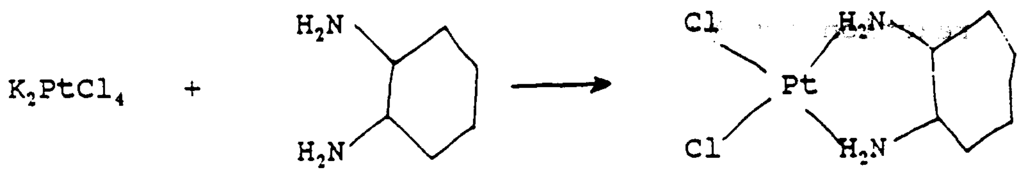

1 2 3

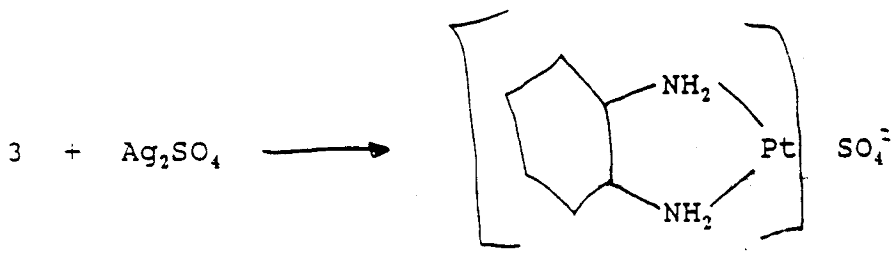

4

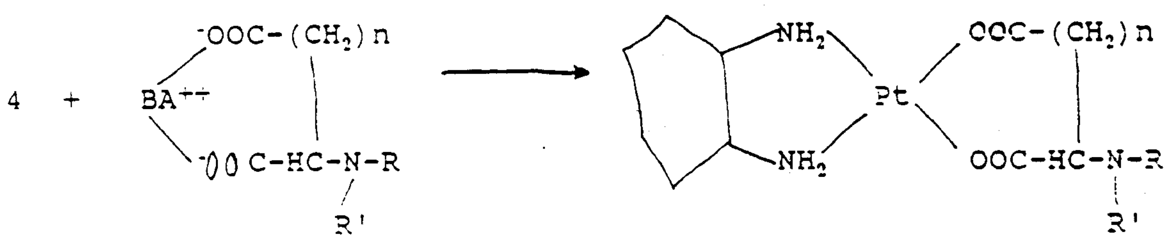

5 6

The following are exemplary of the present invention.

EXAMPLE I

1.2 g of 2-(N-acetylamino)-3,4,6-tri-0-acetyl-2-deoxyglucopyranosyl isothiocyanate in 5 ml acetonitrile is added to a solution of 0.413 g of iminodiacetic acid and 1.12 ml of N,N-diisopropylethylamine in 25 ml of a water-acetonitrile mixture (1:1 V/V). The resulting mixture is stirred until thin layer chromatography ($CHCL_3$:methanol, 9:1) shows complete disappearance of isothiocyanate. Acetonitrile is removed under reduced pressure and the water layer is basified with saturated sodium bicarbonate and is then extracted with $CHCl_3$. The aqueous layer is acidified with 10% HCl and is extracted with ethylacetate. The ethylacetate layer is backwashed with water and is dried over $Na_2SO_4$. The product is further dried through evaporation in vacuo. A intermediate of formula 3 is formed by recrystallizattion from ethylether. 0.52 g of this intermediate is admixed with 0.3 g barium hydroxide $8H_2O$. The resultant is added to 0.4 grams of cis-sulfato-(cyclohexane-1,2-diammine-N,N')-platinum (II) which is already in solution with 20 ml of water. This mixture is stirred at room temperature for 2 hours. Next the barium sulfate is filtered off, and the resulting filtrate is evaporated under reduced pressure to yield a cyclohexane-1,2-diammine-platinum(II) salt/complex of [[[2-(N-acetylamino)-3,4,6-tri-0-acetyl-2-deoxyglucopyranosyl] amino]thioxomethyl]imino-diacetic acid.

EXAMPLE II

The compound of Example I is admixed with an isotonic solution to produce a dosage form suitable for intravenous administration. 130 mg/m$^2$ body surface area of a patient is administered to said patient through intravenous administration over a period of 24 hours.

EXAMPLE III

1.2 g of tetra-0-acetyl-D-mannopyranosyl-isothiocyanate in 5 ml acetonitrile is added to a solution of 0.413 g of iminodiacetic acid and 1.12 ml of N,N-diisopropylethylamine in 25 ml of a water-acetonitrile mixture (1:1 V/V). The resulting mixture is stirred until thin layer chromatography ($CHCl_3$:methanol, 9:1)

shows complete disappearance of isothiocyanate. Acetonitrile is removed under reduced pressure and the water layer is basified with saturated sodium bicarbonate and is then extracted with $CHCl_3$. THe aqueous layer is acidified with 10% HCl and is extracted with ethylacetate. The ethylacetate layer is backwashed with water and is dried over $Na_2SO_4$. The product is further dried through evaporation in vacuo. A intermediate of formula 3 is formed by recrystallization from ethylether.

0.52 g of this intermediate is admixed with 0.3 g barium hydroxide $8H_2O$. The resultant is added to 0.4 gams of cis-sulfato-diammine-platinum(II) which is already in solution with 20 ml of water. This mixture is stirred at room temperature for 2 hours. Next the barium sulfate is filtered off, and the resulting filtrate is evaporated under reduced pressure to yield a diammine-platinum(II) salt/complex of [[[tetra-0-acetyl-D mannopyranosyl]amino] thioxomethyl]imino-diacetic acid.

EXAMPLE IV

The compound of Example III is admixed with an isotonic solution to produce a dosage form suitable for intramuscular administration. 80 mg/m$^2$ body surface area of a patient is administered to said patient through intramuscular administration daily.

EXAMPLE V

1.2 g of tetra-0-acetyl-D-galactopyranosyl-isothiocyanate in 5 ml acetonitrile is added to a solution of a 0.413 g of iminodiacetic acid and 1.12 ml of N,N-diisopropylethylamine in 25 ml of a water-acetonitrile mixture (1:1 V/V). The resulting mixture is stirred until thin layer chromatography ($CHCl_3$:methanol, 9:1) shows complete disappearance of isothiocyanate. Acetonitrile is removed under reduced pressure and the water layer is basified with saturated sodium bicarbonate and is then extracted with $CHCl_3$. The aqueous layer is acidified with 10% HCl and is extracted with ethylacetate. The ethylacetate layer is backwashed with water and is dried over $Na_2SO_4$. The product is further dried through evaporation in vacuo. A intermediate of formula 3 is formed by recrystallization from ethylether.

0.52 g of this intermediate is admixed with 0.3 g barium hydroxide $8H_20$. The resultant is added to 0.4 grams of cis-sulfato cyclohexane-1,2-diammine-N,N'-platinum(II) which is already in solution with 20 ml of water. This mixture is stirred at room temperature for 2 hours. Next the barium sulfate is filtered off, and the resulting filtrate is evaporated under reduced pressure to yield a cyclohexane-1,2-diammine-platinum(II) salt/complex of [[[tetra-0-acetyl galactopyranosyl]amino]thioxomethyl] imino-diacetic acid.

EXAMPLE VI

The compound of Example V is admixed with an isotonic solution to produce a dosage form suitable for intraperitoneal administration. 100 mg/m$^2$ body surface area of a patient is administered to said patient through intraperitoneal administration weekly.

EXAMPLE VII

1.2 g of 3,4,6-tri-0-acetyl-2-(N-acetylamino)-2-deoxy-alpha-D-glucopyranosyl-isothiocyanate in 5 ml acetonitrile is added to a solution of 0.413 g of iminodiacetic acid and 1.12 ml of N,N-diisopropylethylamine in 25 ml of a water-acetonitrile mixture (1:1 V/V). The resulting mixture is stirred until thin layer chromatography ($CHCL_3$:methanol, 9:1) shows complete disappearance of isothiocyanate. Acetonitrile is removed under reduced pressure and the water layer is basified with saturated sodium bicarbonate and is then extracted with $CHCl_3$. The aqueous layer is acidified with 10% HCl and is extracted with ethylacetate. The ethylacetate layer is backwashed with water and is dried over $Na_2SO_4$. The product is further dried through evaporation in vacuo. A intermediate of formula 3 is formed by recrystallization from ethylether.

0.52 g of this intermediate is admixed with 0.3 g barium hydroxide$\bullet 8H_20$. The resultant is added to 0.4 grams of cis-sulfato-amino-methylamino-N,N'-platinum(II) which is already in solution with 20 ml of water. This mixture is stirred at room temperature for 2 hours. Next the barium sulfate is filtered off, and the resulting filtrate is evaporated under reduced pressure to yield a amino-methylamino-N,N'-platinum(II) salt/complex of [[[3,4,6-trio-0-acetyl-2-(N-acetylamino)-2-deoxy-alpha-D-glucopyranosyl] amino]-thioxomethyl]imino diacetic acid.

EXAMPLE VIII

The compound of Example VII is admixed with an hydroxypropylcellulose to form a dosage form suitable for oral administration. 120 mg/m$^2$ body surface area of a patient is administered to said patient through oral administration daily.

EXAMPLE IX

1.2 g of 3,4,6-tri-0-acetyl-2-(N-acetylamino)-2-deoxy-alpha-D-galactopyranosyl-isothiocyanate in 5 ml acetonitrile is added to a solution of 0.413 g of 4-amino-1, 1-cyclohexanedicarboxylic acid and 1.12 ml of diisopropylethylamine in 25 ml of a water-acetonitrile mixture (1:1 V/V). The resulting mixture is stirred until thin layer chromatography ($CHCl_3$:methanol, 9:1) shows complete disappearance of isothiocyanate. Acetonitrile is removed under reduced pressure and the water layer is basified with saturated sodium bicarbonate and is then extracted with $CHCl_3$. The aqueous layer is acidified with 10% HCl and is extracted with ethylacetate. The ethylacetate layer is backwashed with water and is dried over $Na_2SO_4$. The product is further dried through evaporation in vacuo. A intermediate of formula 3 is formed by recrystallization from ethylether.

0.52 g of this intermediate is admixed with 0.3 g barium hydroxide•$8H_20$. The resultant is added to 0.4 grams of cis-sulfato-cyclopentane-1,2-diammine-N,N'-platinum(II) which is already in solution with 20 ml of water. This mixture is stirred at room temperature for 2 hours. Next the barium sulfate is filtered off, and the resulting filtrate is evaporated under reduced pressure to yield a cyclopentane-1,2-diammine-platinum(II) salt/complex of 4-[[[3,4,6-tri-0-acetyl-2-(N-acetylamino)-2-deoxy-alpha-D-galactopyranosyl]amino]-thioxomethyl]-1,1 cyclohexanedicarboxylic acid.

EXAMPLE X

The compound of Example IX is admixed with an isotonic solution to produce a dosage form suitable for intravenous administration. 150 mg/m$^2$ body surface area of a patient is administered to said patient through intravenous administration over a period of 24 hours.

EXAMPLE XI

1.2 g of 2-(N-acetylamino)-3,4,6-tri-0-acetyl-2-deoxyglucopyranosyl isothiocyanate in 5 ml acetonitrile is added to a solution of 0.413 g of 3,3-trimethyleneimino dicarboxylic acid and 1.12 ml of N,N-diisopropylethylamine in 25 ml of a water-acetonitrile mixture (1:1 V/V). The resulting mixture is stirred until thin layer chromatography ($CHCl_3$:methanol, 9:1) shows complete disappearance of isothiocyanate. Acetonitrile is removed under reduced pressure and the water layer is basified with saturated sodium bicarbonate and is then extracted with $CHCl_3$. The aqueous layer is acidified with 10% HCl and is extracted with ethylacetate. The ethylacetate layer is backwashed with water and is dried over $Na_2SO_4$. The product is further dried through evaporation in vacuo. A intermediate of formula 3 is formed by recrystallization from ethylether.

0.52 g of this intermediate is admixed with 0.3 g barium hydroxide•$8H_20$. The resultant is added to 0.4 grams of cis-sulfato-diammine-platinum(II) which is already in solution with 20 ml of water. This mixture is stirred at room temperature for 2 hours. Next the barium sulfate is filtered off, and the resulting filtrate is evaporated under reduced pressure to yield a diammino-platinum(II) salt/complex of 2-(N-acetylamino)-3,4,6-tri-0-acetyl-2-deoxyglucopyranosyl-amino-thioxomethyl-3,3-trimethyleneimino-dicarboxylic acid.

EXAMPLE XII

The compound of Example XI is admixed with an isotonic solution to produce a dosage form suitable for intramuscular administration. 50 mg/m$^2$ body surface area of a patient is administered to said patient through intramuscular administration daily.

EXAMPLE XIII

1.2 g of tetra-0-acetyl-glucopyranosyl-isothiocyanate in 5 ml acetonitrile is added to a solution of 0.413 g of 4-amino-1,1-cyclohexanedicarboxylic acid and 1.12 ml of N,N-diisopropylethylamine in 25 ml of a water-acetonitrile mixture (1:1 V/V). The resulting mixture is stirred until thin layer chromatography

($CHCl_3$:methanol, 9:1) shows complete disappearance of isothiocyanate. Acetonitrile is removed under reduced pressure and the water later is basified with saturated sodium bicarbonate and is then extracted with $CHCl_3$. The aqueous layer is acidified with 10% HCl and is extracted with ethylacetate. The ethylacetate layer is backwashed with water and is dried over $Na_2SO_4$. The product is further dried through evaporation in vacuo. A intermediate of formula 3 is formed by recrystallization from ethylether.

0.52 g of this intermediate is admixed with 0.3 g barium hydroxide•$8H_20$. The resultant is added to 0.4 grams of cis-sulfato-(cyclohexane-1,2-diammine-N,N')-platinum(II) which is already in solution with 20 ml of water. This mixture is stirred at room temperature for 2 hours. Next the barium sulfate is filtered off, and the resulting filtrate is evaporated under reduced pressure to yield a cyclohexane-1,2-diammine-platinum(II) salt/complex of 4-[[(tetra-0-acetyl-alpha-D-glucopyranosyl)amino] thioxomethyl]amino]-1,1-cyclohexanedicarboxylic acid.

EXAMPLE IV

The compound of Example XIII is admixed with an isotonic solution to produce a dosage form suitable for intraperitoneal administration. 150 mg/$m^2$ body surface area of a patient is administered to said patient through intraperitoneal administration every 3 weeks.

EXAMPLE XV

1.2 g of 2-(N-acetylamino)-3,4,6-tri-0-acetyl-2-deoxyglucopyranosyl isothiocyanate in 5 ml acetonitrile is added to a solution of 0.413 g of 3,3-trimethyleneiminodicarboxylic acid and 1.12 ml of diisopropylethylamine in 25 ml of a water-acetonitrile mixture (1:1 V/V). The resulting mixture is stirred until thin layer chromatography ($CHCl_3$:methanol, 9:1) shows complete disappearance of isothiocyanate. Acetonitrile is removed under reduced pressure and the water layer is basified with saturated sodium bicarbonate and is then extracted with $CHCl_3$. The aqueous layer is acidified with 10% HCL and is extracted with ethylacetate. The ethylacetate layer is backwashed with water and is dried over $Na_2SO_4$. The product is further dried through evaporation in vacuo. A intermediate of formula 3 is formed by recrystallization from ethylether.

0.52 g of this intermediate is admixed with 0.3 g barium hydroxide•$8H_20$. The resultant is added to 0.4 grams of cis-sulfato-diammine-platinum(II) which is already in solution with 20 ml of water. This mixture is stirred at room temperature for 2 hours. Next the barium sulfate is filtered off, and the resulting filtrate is evaporated under reduced pressure to yield a diammino-platinum(II) salt/complex of 2-(N-acetylamino)-3,4,6-tri-0-acetyl-2-deoxyglucopyranosyl-amino-thioxomethyl-3,3-trimethyleneimino-dicarboxylic acid.

EXAMPLE XVI

The compound of Example XV is admixed with glycerin monostearate to produce a dosage form suitable for oral administration. 70 mg/$m^2$ body surface area of a patient is administered to said patient through oral administration daily.

EXAMPLE XVII

1.2 g of tetra-0-acetyl-galactopyranosyl-isothiocyanate in 5 ml acetonitrile is added to a solution of 0.413 g of 3,3-trimethyleneiminodicarboxylic acid and 1.12 ml of N,N-diisopropylethylamine in 25 ml of a water-acetonitrile mixture (1:1 V/V). The resulting mixture is stirred until thin layer chromatography ($CHCl_3$:methanol, 9:1) shows complete disappearance of isothiocyanate. Acetonitrile is removed under reduced pressure and the water layer is basified with saturated sodium bicarbonate and is then extracted with $CHCl_3$. The aqueous layer is acidified with 10% HCl and is extracted with ethylacetate. The ethylacetate layer is backwashed with water and is dried over $Na_2SO_4$. The product is further dried through evaporation in vacuo. A intermediate of formula 3 is formed by recrystallization from ethylether.

0.52 g of this intermediate is admixed with 0.3 g barium hydroxide•$8H_20$. The resultant is added to 0.4 grams of cis-sulfato-diammine-platinum(II) which is already in solution with 20 ml of water. This mixture is stirred at room temperature for 2 hours. Next the barium sulfate is filtered off, and the resulting filtrate is evaporated under reduced pressure to yield a diammino-platinum(II) salt/complex of tetra-0-acetyl-galactopyranosyl-aminothioxomethyl-3,3-trimethyleneiminodicarboxylic acid.

EXAMPLE XVIII

The compound of Example XVII is admixed with glycerin monostearate to produce a dosage form suitable for oral administration. 70 mg/$m^2$ body surface area of a patient is administered to said patient through oral administration daily.

EXAMPLE XIX

1.2 g of tetra-0-acetyl-glucopyranosyl isothiocyanate in 5 ml acetonitrile is added to a solution of 0.413 g of 4,4-piperidinedicarboxylic acid and 1.12 ml of N,N-diisopropylethylamine in 25 ml of a water-acetonitrile mixture (1:1 V/V). The resulting mixture is stirred until thin layer chromatography ($CHCl_3$:methanol, 9:1) shows complete disappearance of isothiocyanate. Acetonitrile is removed under reduced pressure and the water layer is basified with saturated sodium bicarbonate and is then extracted with $CHCl_3$. The aqueous layer is acidified with 10% HCl and is extracted with ethylacetate. The ethylacetate layer is backwashed with water and is dried over $Na_2SO_4$. The product is further dried through evaporation in vacuo. A intermediate of formula 3 is formed by recrystallization from ethylether.

0.52 g of this intermediate is admixed with 0.3 g barium hydroxide•$8H_20$. The resultant is added to 0.4 grams of cis-sulfato-cyclohexane-1,2-diammine-platinum(II) which is already in solution with 20 ml of water. This mixture is stirred at room temperature for 2 hours. Next the barium sulfate is filtered off, and the resulting filtrate is evaporated under reduced pressure to yield a 1,2-cyclohexane-diammino-platinum(II) salt/complex of 4-[[[(tetra-0-acetyl-glucopyranosyl)-amino]thioxomethyl] amino]-4,4-piperidinedicarboxylic acid.

EXAMPLE XX

The compound of Example XV is admixed with hydroxypropylcellulose to produce a dosage form suitable for oral administration. 100 mg/$m^2$ body surface area of a patient is administered to said patient through oral administration daily.

EXAMPLE XXI

The cyclohexane-1,2-diammine-platinum(II) salt of 2-(acetylamino)-3,4,6-tri-0-acetyl-2-deoxyglucopyranosylamino-thioxomethyl-imino diacetic acid and cisplatin were tested against murine P388 leukemia. The murine P388 leukemia system is known to be sensitive to cisplatin. The leukemia was maintained intraperitoneally in female DBA/2 mice.

Prior to administration, cisplatin was dissolved in ethanol. The solution was then adjusted to 5% ethanol, 95% sterile water. The cyclohexane-1,2-diammine-platinum(II) salt of 2-(acetylamino)-3,4,6-tri-0-acetyl-2-deoxyglucopyranosylamino-thioxomethyl-imino diacetic acid was dissolved in sterile water at 4 degrees celsius immediately prior to administration.

Each compound was administered intraperitoneally to groups of $CD2F_1$ male mice on day 1 after intraperitoneal implantation of 1 x $10^6$ P388 leukemia cells. P388 antileukemic activity for each compound was assessed by mean survival days and percentage increased life span (% ILS).

% ILS is calculated as follows:

$$\%ILS = (T-C)/C \times 100$$

wherein T is the mean survival days of the treated mice and C is the mean survival days of the untreated mice. The results of the experimentation are shown in the following table.

TABLE 1

| Compound | Dose | %ILS | Mean Survival (days) |
|---|---|---|---|
| cisplatin | 10 mg/kg | 83 | 17.4 |
| invention | 100 mg/kg | 80 | 17u.1 |

EXAMPLE XXII

Preparation of Dicarboxylic Acid Ligand.

3,4,6-tri-0-acetyl-2-(acetylamino)-2-deoxyglucopyranosyl isothiocyanate is added to a solution of aspartic acid and N,N-diisopropylethylamine in a mixture of water-acetonitrile. The mixture is stirred at room temperature in the dark until thin-layer chromatography (chloroform: methanol 10:1) indicates reaction completion. Acetonitrile is removed and the water layer is basified with 10% $NaHCO_3$. The basic solution is extracted with 2X75 ml $CH_2CL_2$, is acidified with 10% HCl and is extracted with 2X100 ml of ethyl acetate. The ethyl acetate layer is dried over anhydrous $Na_2SO_4$ and is evaporated to dryness to give 2-[[[3,4,6-tri-0-acetyl-2-(N-acetylamino)-2-deoxy-alpha-D-glucopyranosyl]amino]thioxomethyl]amino]butanedioic acid. M.p. 124-126.

EXAMPLE XXIII

Preparation of Cis-Sulfato-DACH-Platinum (II)

To a freshly prepared solution of $K_2PtCl_4$ is added an equimolar amount of 1,2-cyclohexanediamine in distilled water. This mixture is allowed to react at room temperature in a nitrogen atmosphere protected from light for 8 hours. The precipitate is washed successively with 10% HCl, $H_20$, ethanol, acetone and ether. After drying in vacuo over $P_20_5$ over night, the cis-dichloro-(1,2-cyclohexanediammine)-platinum (II) is stirred with an equimolar amount of silver sulfate is distilled, degassed water under nitrogen atmosphere for 36 hours in the dark. The silver chloride precipitate is removed and the filtrate is freeze dried to give cis-sulfato-DACH-platinum (II).

EXAMPLE XXIV

Cis-sulfato-DACH-platinum (II) is prepared in accordance with Example XXIII. Barium L-aspartate is prepared in situ using the appropriate dicarboxylic ligand. The cis-sulfato-DACH-platinum (II) and barium L-aspartate are combined and are agitated in a nitrogen atmosphere in the dark for 2 hours. Barium sulfate precipitate is filtered off and the filtrate is concentrated to about 2 ml. Acetone is added to the concentrated solution resulting in a white precipitate. This precipitate is further purified by successive washing with acetone and ether. The resulting product is (L-aspartato-0,0')-(1,2-cyclohexanediammine-N,N')-platinum (II). (Turned brown at 240, decomposed at 280).

EXAMPLE XXV

5.1 mmol Cis-Pt $(NH_3)I_2$ is added to 5 mmol $Ag_2SO4$ in 200 ml degassed, distilled water and is stirred in the dark at room temperature for 4 hours. AgI precipitate is filtered off and the filtrate is concentrated to about 80 ml. A solution of 5 mmol barium 2-[[[3,4,6-tri-0-acetyl-2-(N-acetylamino-2-deoxy-alpha-D-glucopyranosyl]amino]thioxo methyl]amino]butanedioate is prepared in situ using the appropriate dicarboxylic ligand which is prepared in accordance with Example XXII. The barium compound is combined with the cis-diammine sulfato-platinum (II) solution and the mixture is agitated at room temperature for 2 hours. Barium sulfate precipitate is filtered off and the filtrate is concentrated to about 1 ml. Acetone is added to the concentrated solution resulting in a yellow precipitate. This precipitate is further purified by successive washing with acetone and ether. The resulting product is Diammine (2-[[[[3,4,6-tri-0-acetyl-2-(N-acetylamino)-2-deoxy-alpha-D-glucopyranosyl]amino]thioxomethyl] amino]butanedioate-0,0']-platinum (II). (Decomposed at 190).

EXAMPLE XXVI

Cis-sulfato-DACH-platinum (II) is prepared in accordance with Example XXIII. Barium 2-[[[3,4,6-tri-0-acetyl-2-(N-acetylamino)-2-deoxy-alpha-D-glucopyranosyl]amino]thioxomethyl]amino]butanedioate is prepared in situ using the appropriate dicarboxylic ligand which is prepared in accordance with Example XXII. The cis-sulfato-DACH-platinum (II) and barium (2-[[[3,4,6-tri-0-acetyl-2-(N-acetylamino)-2-deoxy-alpha-D-glucopyranosyl]amino]thioxomethyl]amino]butanedioate are combined and are agitated in a nitrogen atmosphere in the dark for 2 hours. Barium sulfate precipitate is filtered off and the filtrate is concentrated to about 2 ml. Acetone is added to the concentrated solution resulting in a white precipitate. This precipitate is

further purified by successive washing with acetone and ether. The resulting product is 2-[[[[3, 4,6-tri-0-acetyl-2-(N-acetylamino)-2-deoxy-alpha-D-glucopyranosyl]amino]thioxomethyl]amino]butanedioate-0, 0']-(1,2-cyclohexanediammine)-N,N'-platinum (II). Turned brown at 200, decomposed at 260.

EXAMPLE XXVII

The compound 2-[[[3,4,6-tri-0-acetyl-2-(N-acetylamino)-2-deoxy-alpha-D-glucopyranosyl]amino]-thioxomethyl]amino] butanedioato-0,0']-[1,2-cyclohexandeiammine-N,N']platinum (II) and cisplatin were tested against murine P388 leukemia. The murine P388 leukemia system is known to be sensitive to cisplatin. The leukemia was maintained intraperitoneally in female DBA/2 mice.

Prior to administration, cisplatin was dissolved in sterile saline (0.85% sodium chloride). The compound 2-[[[3,4,6-tri-0-acetyl-2-(N-acetylamino)-2-deoxy-alpha-D-glucopyranosyl]amino]thioxomethyl]amino]-butanedioato-0,0']-[1,2-cyclohexanediammine-N,N']platinum (II) was dissolved in sterile water at 4°C immediately prior to administration.

Each compound was administered intraperitoneally to groups of $CD2F_1$ male mice on day 1 after intraperitoneal implantation of 1 x $10^6$ P388 leukemia cells. P388 antileukemic activity for each compound was assessed by mean survival days and percentage increased life span (%ILS).

%ILS is calculated as follows:

$$\% \text{ ILS} = (T-C)/C \times 100$$

wherein T is the mean survival days of the treated mice and C is the mean survival days of the untreated mice. The results of the experimentation are shown in the following table.

TABLE 2

| Compound | Dose | %ILS | Mean Survival (days) |
|---|---|---|---|
| cisplatin | 10 mg/kg | 96 | 15.7 |
| invention | 400 mg/kg | 76 | 14.1 |
| invention | 800 mg/kg | toxic | - |

## Claims

**1.** A compound of the formula:

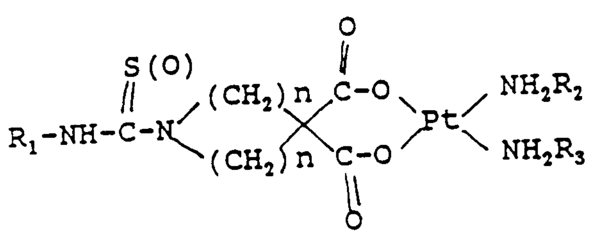

wherein n is 1 or 2; $R_1$ is a mono or disaccharide or derivative thereof; each of $R_2$ and $R_3$ is independently selected from the group consisting of hydrogen or $C_{1-4}$ alkyl or $R_2$ and $R_3$ together are linked to adjacent carbon atoms on a five or six membered ring structure

or a pharmaceutically acceptable salt thereof.

**2.** A compound of claim 1, wherein $R_1$ is a mono or disaccharide or derivative thereof selected from the group consisting of glucose, galactose, mannose, glucosamine and galactosamine and derivatives thereof.

**3.** A compound of claim 1, wherein $R_2$ and $R_3$ are hydrogen.

4. A compound of claim 1, wherein $R_2$ and $R_3$ together are linked to adjacent carbon atoms on a five or six membered ring structure.

5. A compound of the formula:

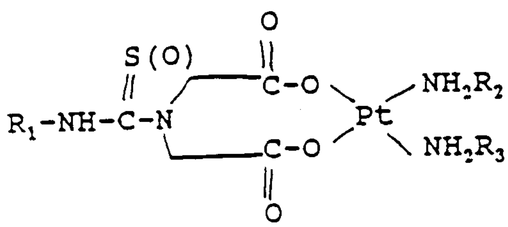

wherein $R_1$ is a mono or disaccharide or a derivative thereof, $R_2$ and $R_3$ are selected from the group consisting of hydrogen $C_{1-4}$ alkyl or $R_2$ and $R_3$ together are linked to adjacent carbon atoms on a five or six membered ring structure
or a pharmaceutically acceptable salt thereof.

6. A compound of claim 5, wherein $R_1$ is a mono or disaccharide or derivative thereof selected from the group consisting of glucose, galactose, mannose, glucosamine and galactosamine and derivatives thereof.

7. A compound of claim 5, wherein $R_2$ and $R_3$ are hydrogen.

8. A compound of claim 5, wherein $R_2$ and $R_3$ together are linked to adjacent carbon atoms on a five or six membered ring structure.

9. A compound of claim 1, wherein the compound is of the formula:

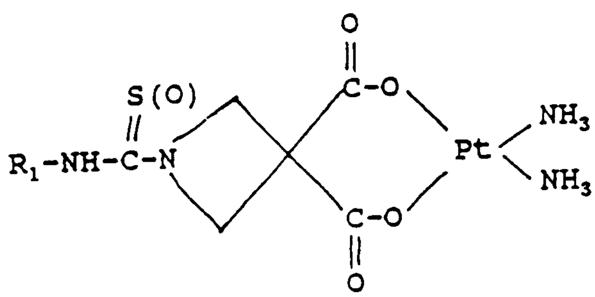

and $R_1$ is selected from the group comprising glucose, mannose, galactose, glucosamine, galactosamine and derivatives thereof.

10. A compounds of claim 1, wherein the compound is of the formula:

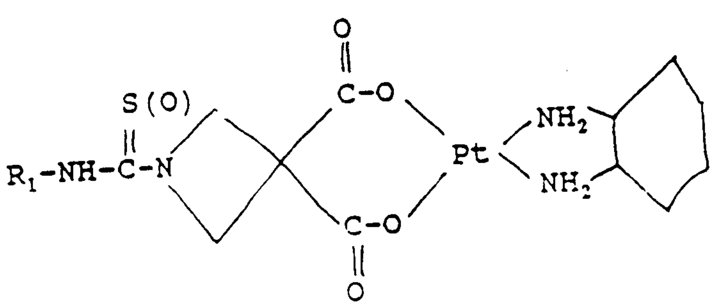

and $R_1$ is selected from the group comprising glucose, mannose, galactose, glucosamine, galactosamine and derivatives thereof.

**11.** A compound of claim 1, wherein the compound is of the formula:

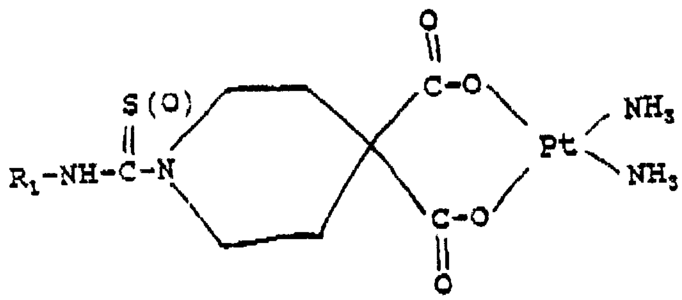

and $R_1$ is selected from the group comprising glucose, mannose, galactose, glucosamine, galactosamine and derivatives thereof.

**12.** A compound of claim 1, wherein the compound is of the formula;

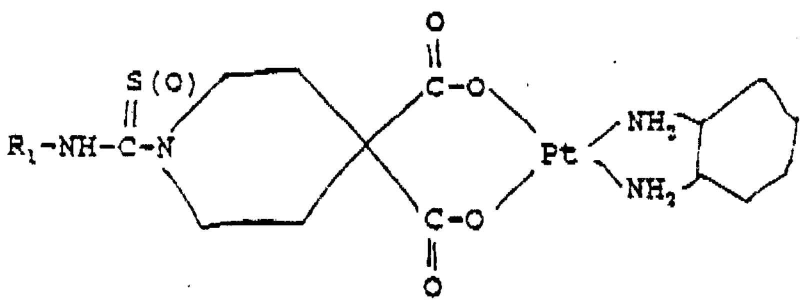

and $R_1$ is selected from the group comprising glucose, mannose, galactose, glucosamine, galactosamine and derivatives thereof.

**13.** A compound of claim 5, wherein the compound is of the formula:

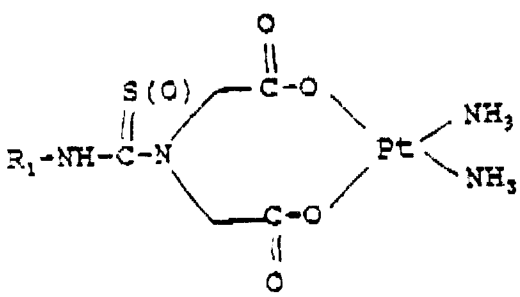

and $R_1$ is selected from the group comprising glucose, mannose, galactose, glucosamine, galactosamine and derivatives thereof.

**14.** A compound of claim 5, wherein the compound is of the formula:

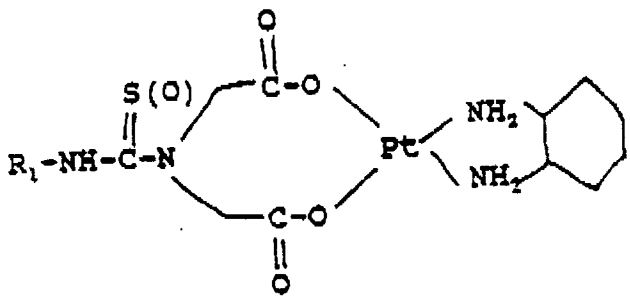

and $R_1$ is selected from the group comprising glucose, mannose, galactose, glucosamine, galactosamine and derivatives thereof.

**15.** A compound of claim 5, wherein the compound is of the formula:

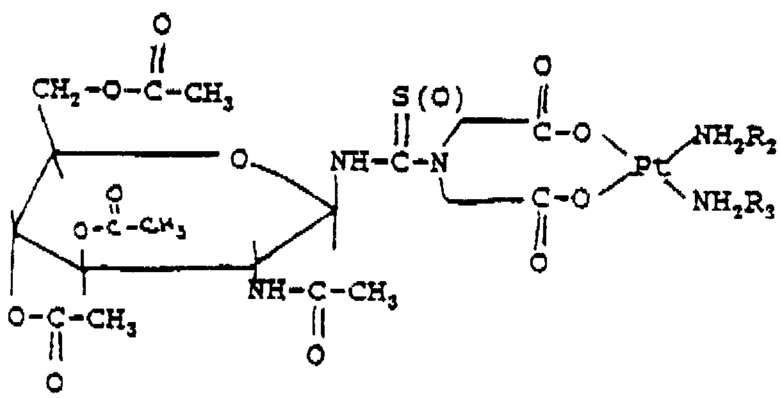

wherein $R_2$ and $R_3$ are as defined above.

**16.** A compound of the formula:

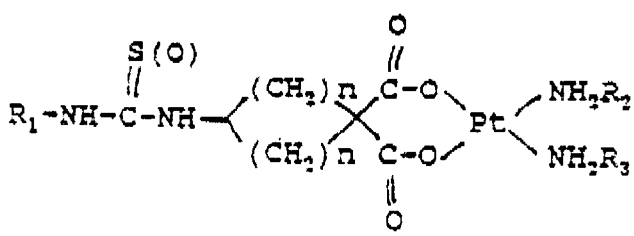

wherein n is 1 or 2; $R_1$ is a mono or disaccharide or derivative thereof; each of $R_2$ and $R_3$ is independently selected from the group consisting of hydrogen or $C_{1-4}$ alkyl, or $R_2$ and $R_3$ together are linked to adjacent carbon atoms on a five or six membered ring structure

or a pharmaceutically acceptable silt thereof.

**17.** A compound of claim 16, wherein said compound is of the formula:

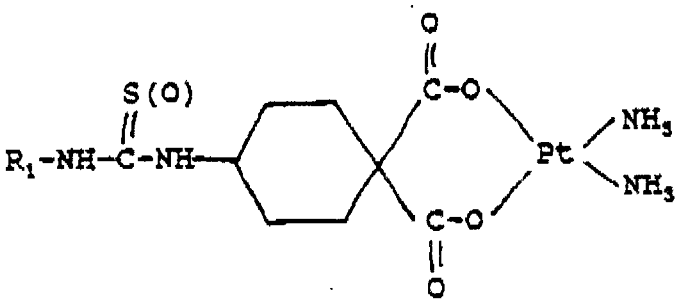

and $R_1$ is selected from the group comprising glucose, mannose, galactose, glucosamine, galactosamine and derivatives thereof.

**18.** A compound of claim 16, wherein said compound is of the formula:

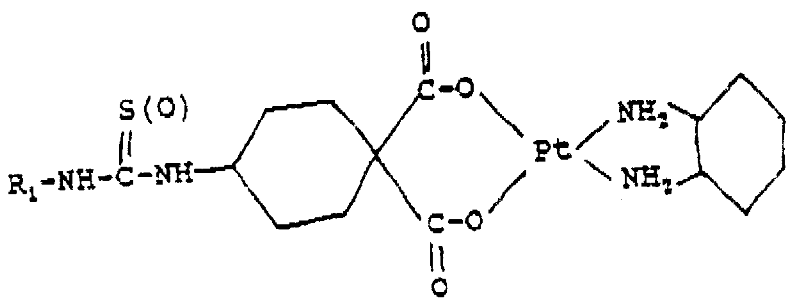

and $R_1$ is selected from the group comprising glucose, mannose, galactose, glucosamine, galactosamine and derivatives thereof.

**19.** A compound of the formula

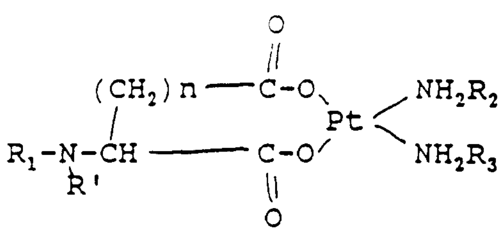

wherein n is 0 or 1, $R_1$ is selected from the group consisting of hydrogen, a mono or disaccharide or a derivative thereof linked to the nitrogen atom by a -NHCO- amide moiety, an -NHCS- thioamide moiety or a -CO- carbonylmoiety, R' is selected from the group consisting of hydrogen and $C_{1-4}$ alkyl, and $R_2$ and $R_3$ are selected from the group consisting of hydrogen, $C_{1-4}$ alkyl or $R_2$ and $R_3$ together are linked to adjacent carbon atoms on a four, five or six membered ring structure, or $R_2$ and $R_3$ together form a fused or bicyclic ring with adjacent carbon atoms; with the proviso that R' and $R_1$ cannot both be hydrogen when n = 0.

or a pharmaceutically acceptable salt thereof.

**20.** A compound of claim 19, wherein $R_1$ is a mono or disaccharide or derivative thereof selected from the group consisting of glucose, galactose, mannose, glucosamine and galactosamine and derivatives thereof.

**21.** A compound of claim 19, wherein $R_2$ and $R_3$ are hydrogen.

**22.** A compound of claim 19, wherein $R_2$ and $R_3$ together are linked to adjacent carbon atoms on a four, five or six membered ring structure.

**23.** A compound of claim 19, wherein $R_2$ and $R_3$ together form a fused or bicyclic ring with adjacent carbon atoms.

**24.** A compound of claim 19, wherein $R_2$ and $R_3$ together form a group of the following formula:

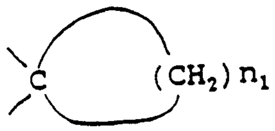

wherein $n_1$ is selected from 1, 2, 3, 4, 5 or 6.

**25.** A compound of claim 19, wherein $R_2$ and $R_3$ together form a group of the following formula:

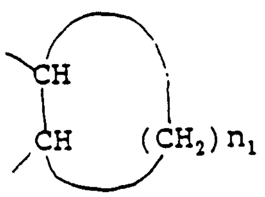

wherein $n_1$ is selected from 1, 2, 3, 4, 5 or 6.

**26.** A compound of claim 19, wherein said compound is of the formula

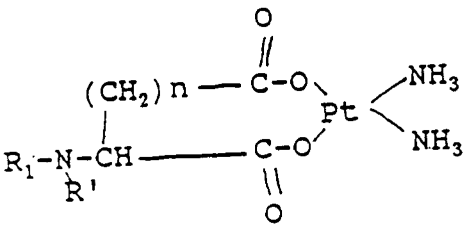

and $R_1$ and R' are defined as in claim 19.

**27.** A compound of claim 19, wherein said compound is of the formula

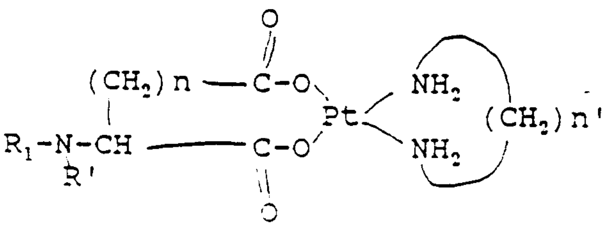

and $R_1$ and R' are defined as in claim 19 and n' is 1, 2 or 3.

**28.** A compound of claim 19, wherein said compound is (L-aspartato-O,O')-(1,2-cyclohexanediammine-N,N')-platinum (II).

**29.** A compound of claim 19, wherein said compound is diammine-2-[[[[3,4,6-tri-O-acetyl-2-(N-acetylamino)-2-deoxy-alpha-D-glucopyranosyl]amino]thioxomethyl]amino] butanedioato- O,O']-platinum (II).

**30.** A compound of claim 19, wherein said compound is 2-[[[[3,4,6-tri-O-acetyl-2-(N-acetylamino)-2-deoxy-alpha-D-glucopyranosyl]amino]thioxomethyl]amino]butanedioato-O,O']-(1,2-cyclohexanediammine-N,N')-platinum (II).

**31.** A pharmaceutical composition comprising a compound according to any one of claims 1 to 30 as active ingredient.

**Patentansprüche**

**1.** Verbindung der Formel

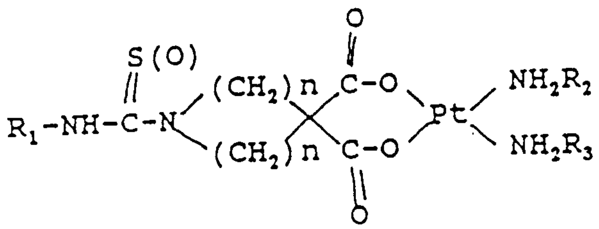

worin bedeuten:

n die Zahl 1 oder 2;

$R_1$ ein Mono- oder Disaccharid oder ein Derivat davon;

$R_2$ und $R_3$ unabhängig voneinander jeweils ausgewählt werden aus der Gruppe, bestehend aus Wasserstoff oder $C_1$-$C_4$-Alkyl, oder $R_2$ und $R_3$ gemeinsam an benachbarte Kohlenstoffatome gebunden sind an einer 5- oder 6-gliedrigen Ringstruktur,

oder ein pharmazeutisch akzeptables Salz davon.

**2.** Verbindung nach Anspruch 1, worin $R_1$ steht für ein Mono- oder Disaccharid oder ein Derivat davon, ausgewählt aus der Gruppe, die besteht aus Glucose, Galactose, Mannose, Glucosamin und Galactosamin, und Derivaten davon.

**3.** Verbindung nach Anspruch 1, worin $R_2$ und $R_3$ Wasserstoff bedeuten.

**4.** Verbindung nach Anspruch 1, worin $R_2$ und $R_3$ gemeinsam an benachbarte Kohlenstoffatome gebunden sind an einer 5- oder 6-gliedrigen Ringstruktur.

**5.** Verbindung der Formel

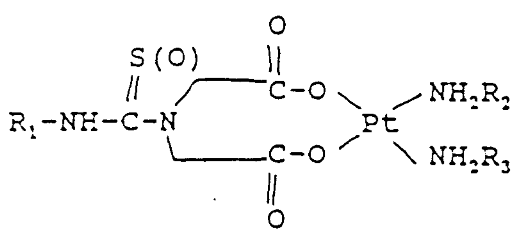

worin bedeuten:

$R_1$ ein Mono- oder Disaccharid oder ein Derivat davon,

$R_2$ und $R_3$ ausgewählt werden aus der Gruppe, die besteht aus Wasserstoff oder $C_1$-$C_4$-Alkyl oder $R_2$ und $R_3$ gemeinsam an benachbarte Kohlenstoffatome gebunden sind an einer 5- oder 6-gliedrigen Ringstruktur,

oder ein pharmazeutisch akzeptables salz davon.

6. Verbindung nach Anspruch 5, worin $R_1$ steht für ein Mono- oder Disaccharid oder ein Derivat davon, ausgewählt aus der Gruppe, die besteht aus Glucose, Galactose, Mannose, Glucosamin und Galactosamin, und Derivaten davon.

7. Verbindung nach Anspruch 5, worin $R_2$ und $R_3$ Wasserstoff bedeuten.

8. Verbindung nach Anspruch 5, worin $R_2$ und $R_3$ gemeinsam an benachbarte Kohlenstoffatome gebunden sind an einer 5- oder 6-gliedrigen Ringstruktur.

9. Verbindung nach Anspruch 1, worin die Verbindung die Formel hat

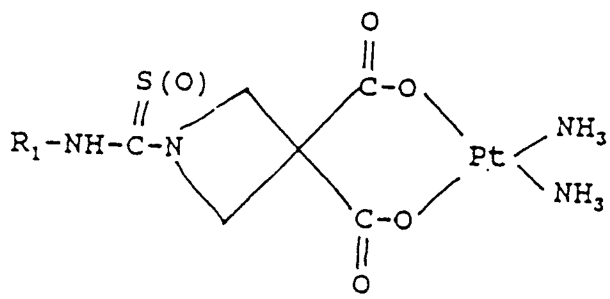

und $R_1$ ausgewählt wird aus der Gruppe, die besteht aus Glucose, Mannose, Galactose, Glucosamin, Galactosamin und Derivaten davon.

10. Verbindung nach Anspruch 1, worin die Verbindung die Formel hat

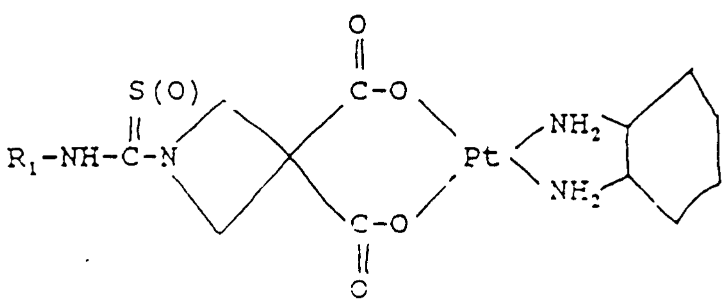

und $R_1$ ausgewählt wird aus der Gruppe, die besteht aus Glucose, Mannose, Galactose, Glucosamin, Galactosamin und Derivaten davon.

11. Verbindung nach Anspruch 1, worin die Verbindung die Formel hat

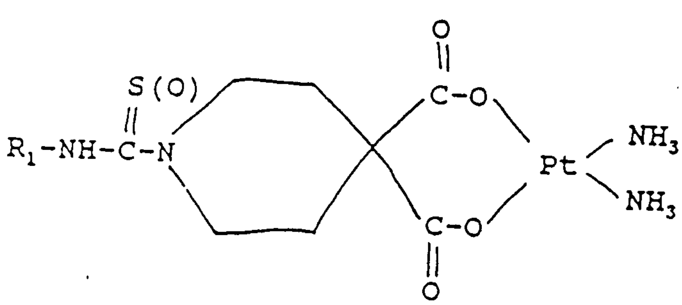

und $R_1$ ausgewählt wird aus der Gruppe, die besteht aus Glucose, Mannose, Galactose, Glucosamin, Galactosamin und Derivaten davon.

**12.** Verbindung nach Anspruch 1, worin die Verbindung die Formel hat

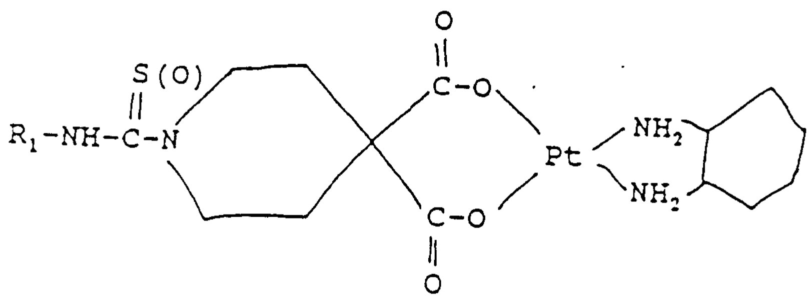

und $R_1$ ausgewählt wird aus der Gruppe, die besteht aus Glucose, Mannose, Galactose, Glucosamin, Galactosamin und Derivaten davon.

**13.** Verbindung nach Anspruch 5, worin die Verbindung die Formel hat

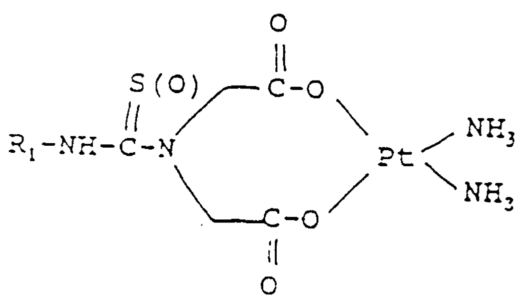

und $R_1$ ausgewählt wird aus der Gruppe, die besteht aus Glucose, Mannose, Galactose, Glucosamin, Galactosamin und Derivaten davon.

**14.** Verbindung nach Anspruch 5, worin die Verbindung die Formel hat

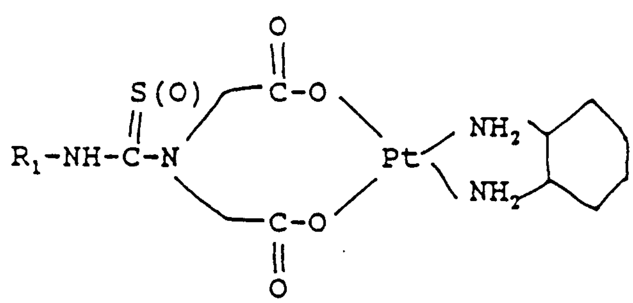

und $R_1$ ausgewählt wird aus der Gruppe, die besteht aus Glucose, Mannose, Galactose, Glucosamin, Galactosamin und Derivaten davon.

**15.** Verbindung nach Anspruch 5, worin die Verbindung die Formel hat

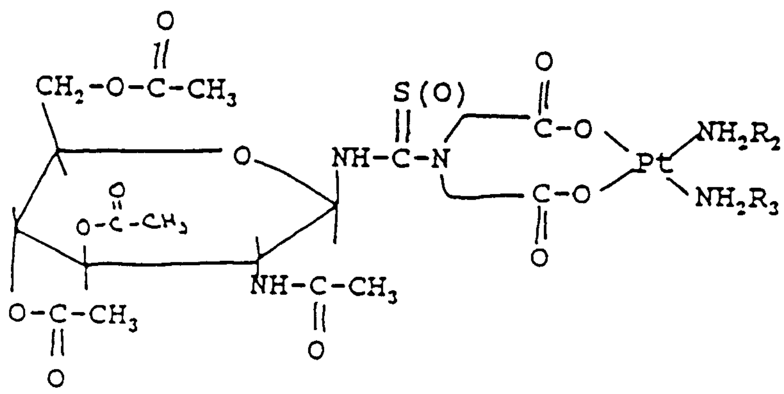

worin $R_2$ und $R_3$ wie oben definiert sind.

**16.** Verbindung der Formel

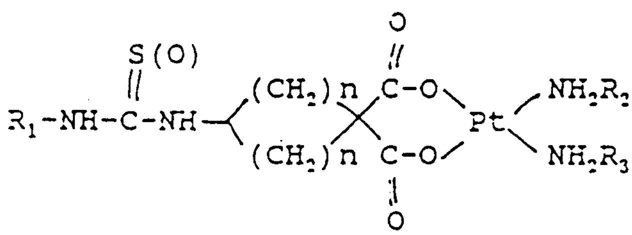

worin bedeuten:

| | |
|---|---|
| n | die Zahl 1 oder 2, |
| $R_1$ | ein Mono- oder Disaccharid oder ein Derivat davon; |
| $R_2$ und $R_3$ | unabhängig voneinander jeweils ausgewählt werden aus der Gruppe, die besteht aus Wasserstoff oder $C_1$-$C_4$-Alkyl oder $R_2$ und $R_3$ gemeinsam an benachbarte Kohlenstoffatome gebunden sind an einer 5- oder 6-gliedrigen Ringstruktur, |

oder ein pharmazeutisch akzeptables Salz davon.

**17.** Verbindung nach Anspruch 16, worin die Verbindung die Formel hat

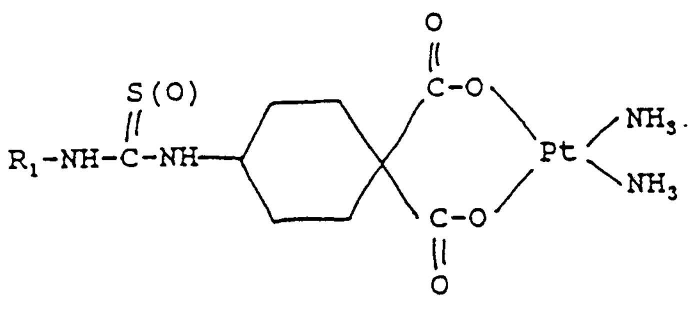

und $R_1$ ausgewählt wird aus der Gruppe, die besteht aus Glucose, Mannose, Galactose, Glucosamin, Galactosamin und Derivaten davon.

**18.** Verbindung nach Anspruch 16, worin die Verbindung die Formel hat

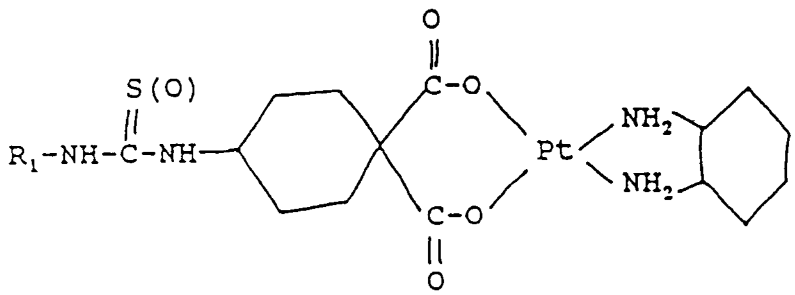

und $R_1$ ausgewählt wird aus der Gruppe, die besteht aus Glucose, Mannose, Galactose, Glucosamin, Galactosamin und Derivaten davon.

**19.** Verbindung der Formel

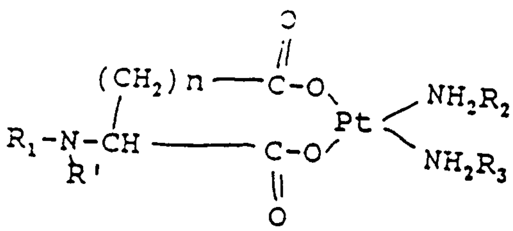

worin bedeuten:

| | |
|---|---|
| n | die Zahl 0 oder 1, |
| $R_1$ | ausgewählt wird aus der Gruppe, die besteht aus Wasserstoff, einem Mono- oder Disaccharid oder einem Derivat davon, das an das Stickstoffatom gebunden ist über einen -NHCO- Amid-Rest, einen -NHCS- Thioamid-Rest oder einen -CO- Carbonyl-Rest, |
| R' | ausgewählt wird aus der Gruppe, die besteht aus Wasserstoff und $C_1$-$C_4$-Alkyl und |
| $R_2$ und $R_3$ | ausgewählt werden aus der Gruppe, die besteht aus Wasserstoff oder $C_1$-$C_4$-Alkyl oder $R_2$ und $R_3$ gemeinsam an benachbarte Kohlenstoffatome gebunden sind an einer 4-, 5- oder 6-gliedrigen Ringstruktur oder $R_2$ und $R_3$ gemeinsam zusammen mit benachbarten Kohlenstoffatomen einen kondensierten oder bicyclischen Ring bilden; |

mit der Maßgabe, daß R' und $R_1$ nicht beide Wasserstoff sein können, wenn n = 0,

oder ein pharmazeutisch akzeptables Salz davon.

**20.** Verbindung nach Anspruch 19, worin $R_1$ steht für ein Mono- oder Disaccharid oder ein Derivat davon, ausgewählt aus der Gruppe, die besteht aus Glucose, Galactose, Mannose, Glucosamin und Galactosamin und Derivaten daovn.

**21.** Verbindung nach Anspruch 19, worin $R_2$ und $R_3$ Wasserstoff bedeuten.

**22.** Verbindung nach Anspruch 19, worin $R_2$ und $R_3$ gemeinsam an benachbarte Kohlenstoffatome gebunden sind an einer 4-, 5- oder 6-gliedrigen Ringstruktur .

**23.** Verbindung nach Anspruch 19, worin $R_2$ und $R_3$ gemeinsam zusammen mit Kohlenstoffatomen einen kondensierten oder bicyclischen Ring bilden.

**24.** Verbindung nach Anspruch 19, worin $R_2$ und $R_3$ gemeinsam eine Gruppe der folgenden Formel bilden

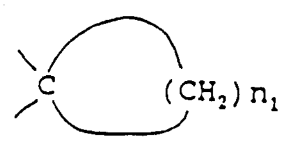

worin $n_1$ ausgewählt wird aus 1, 2, 3, 4, 5 oder 6.

**25.** Verbindung nach Anspruch 19, worin $R_2$ und $R_3$ gemeinsam eine Gruppe der folgenden Formel bilden:

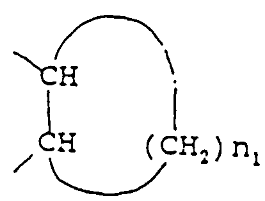

worin $n_1$ ausgewählt wird 1, 2, 3, 4, 5 oder 6.

**26.** Verbindung nach Anspruch 19, worin diese Verbindung die Formel hat

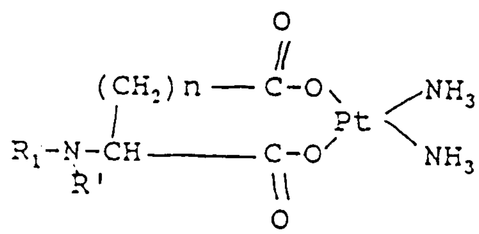

und $R_1$ und R' wie in Anspruch 19 definiert sind.

**27.** Verbindung nach Anspruch 19, worin diese Verbindung die Formel hat

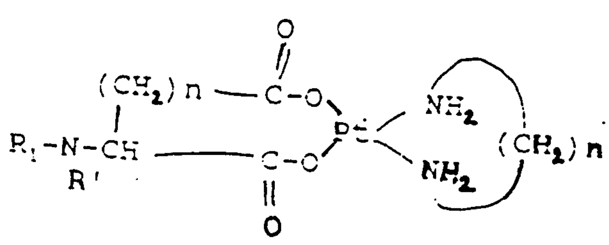

und $R_1$ und R' wie in Anspruch 19 definiert sind und n' für 1, 2 oder 3 steht.

**28.** Verbindung nach Anspruch 19, wobei es sich bei dieser Verbindung handelt um

(L-Aspartato-O,O')-(1,2-cyclohexandiammin-N,N')platin(II).

**29.** Verbindung nach Anspruch 19, wobei es sich bei dieser Verbindung handelt um

Diammin-2-[[[[3,4,6-tri-O-acetyl-2-(N-acetylamino)-2-deoxy-α-D-glucopyranosyl]amino]thioxomethyl]-amino]butandioato-O,O']platin (II).

**30.** Verbindung nach Anspruch 19, wobei es sich bei dieser Verbindung handelt um

2-[[[[3,4,6-Tri-O-acetyl-2-(N-acetylamino)-2-deoxy-α-D-glucopyranosyl]amino]-thioxomethylaminobutandioato-O,O'-(1,2-cyclohexandiammin-N,N')platin (II).

**31.** Pharmazeutische Zusammensetzung, die eine Verbindung nach einem der Ansprüche 1 bis 30 als aktiven Bestandteil (Wirkstoff) enthält.

## Revendications

**1.** Composé de formule :

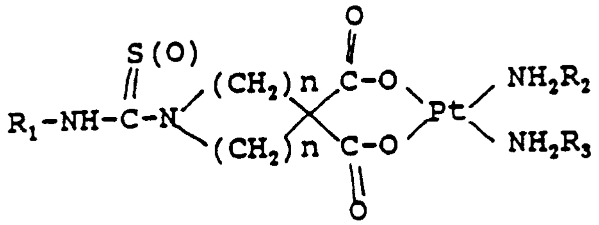

dans laquelle n est égal à 1 ou 2; $R_1$ est un mono- ou un di-saccharide, ou un dérivé de ceux-ci; chacun des radicaux $R_2$ et $R_3$ est choisi indépendamment dans le groupe constitué par un hydrogène ou un groupe alkyle en $C_1$-$C_4$, ou $R_2$ et $R_3$ sont liés ensemble à des atomes de carbone adjacents sur une structure cyclique à cinq ou six chaînons

ou sel pharmaceutiquement acceptable de ce composé.

**2.** Composé selon la revendication 1, dans lequel $R_1$ est un mono- ou un di-saccharide, ou un dérivé de ceux-ci, choisi dans le groupe constitué par le glucose, le galactose, le mannose, la glucosamine et la galactosamine, et des dérivés de ceux-ci.

**3.** Composé selon la revendication 1, dans lequel $R_2$ et $R_3$ sont des atomes d'hydrogène.

**4.** Composé selon la revendication 1, dans lequel $R_2$ et $R_3$ ensemble sont liés à des atomes de carbone adjacents sur une structure cyclique à cinq ou six chaînons.

**5.** Composé de formule :

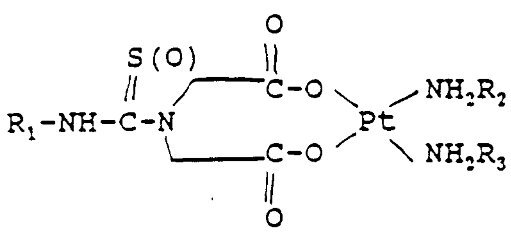

dans laquelle $R_1$ est un mono- ou un di-saccharide, ou un dérivé de ceux-ci, $R_2$ et $R_3$ sont choisis dans le groupe constitué par un hydrogène, un groupe alkyle en $C_1$-$C_4$, ou $R_2$ et $R_3$ ensemble sont liés à des atomes de carbone adjacents sur une structure cyclique à cinq ou six chaînons,

ou sel pharmaceutiquement acceptable de ce composé.

**6.** Composé selon la revendication 5, dans lequel $R_1$ est un mono- ou un di-saccharide, ou un dérivé de ceux-ci, choisi dans le groupe constitué par le glucose, le galactose, le mannose, la glucosamine et la galactosamine, et des dérivés de ceux-ci.

**7.** Composé selon la revendication 5, dans lequel $R_2$ et $R_3$ sont des atomes d'hydrogène.

**8.** Composé selon la revendication 5, dans lequel $R_2$ et $R_3$ ensemble sont liés à des atomes de carbone adjacents sur une structure cyclique à cinq ou six chaînons.

**9.** Composé selon la revendication 1, dans lequel le composé a pour formule :

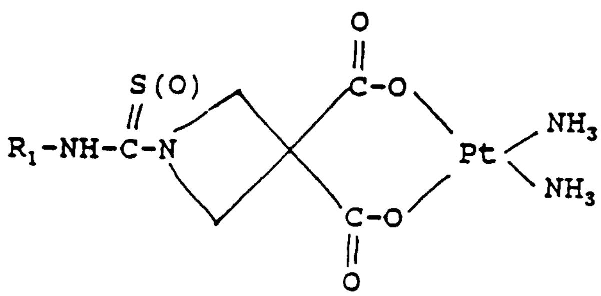

et $R_1$ est choisi dans le groupe comprenant le glucose, le mannose, le galactose, la glucosamine, la galactosamine et des dérivés de ceux-ci.

**10.** Composé selon la revendication 1, dans lequel le composé a pour formule :

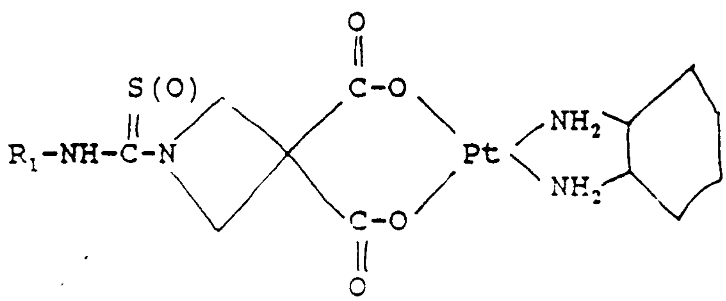

et $R_1$ est choisi dans le groupe comprenant le glucose, le mannose, le galactose, la glucosamine, la galactosamine et des dérivés de ceux-ci.

**11.** Composé selon la revendication 1, dans lequel le composé a pour formule :

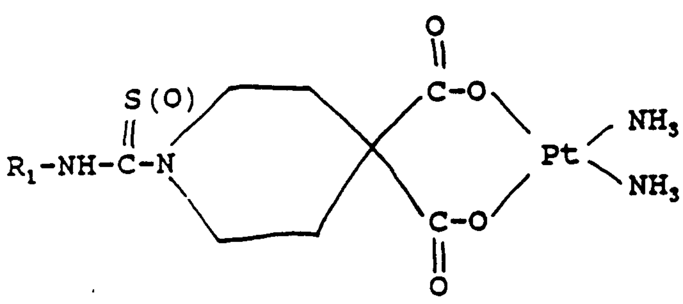

et $R_1$ est choisi dans le groupe comprenant le glucose, le mannose, le galactose, la glucosamine, la galactosamine et des dérivés de ceux-ci.

**12.** Composé selon la revendication 1, dans lequel le composé a pour formule :

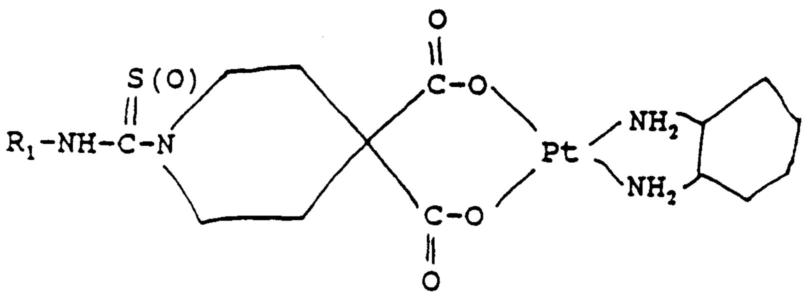

et $R_1$ est choisi dans le groupe comprenant le glucose, le mannose, le galactose, la glucosamine, la galactosamine et des dérivés de ceux-ci.

**13.** Composé selon la revendication 5, dans lequel le composé a pour formule :

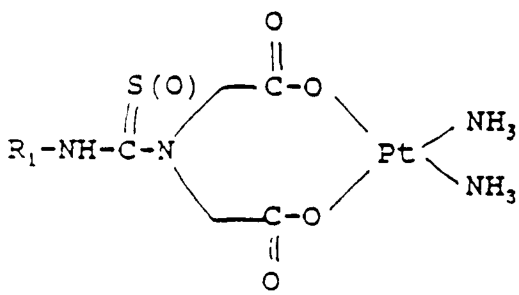

et $R_1$ est choisi dans le groupe comprenant le glucose, le mannose, le galactose, la glucosamine, la galactosamine et des dérivés de ceux-ci.

**14.** Composé selon la revendication 5, dans lequel le composé a pour formule :

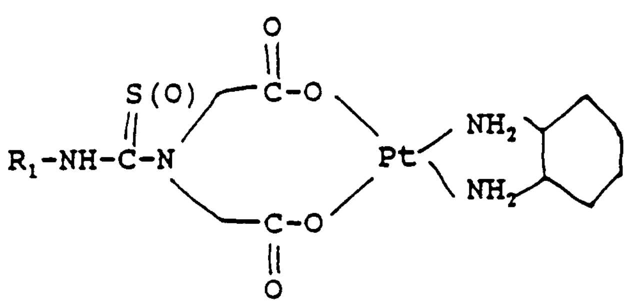

et $R_1$ est choisi dans le groupe comprenant le glucose, le mannose, le galactose, la glucosamine, la galactosamine et des dérivés de ceux-ci.

**15.** Composé selon la revendication 5, dans lequel le composé a pour formule :

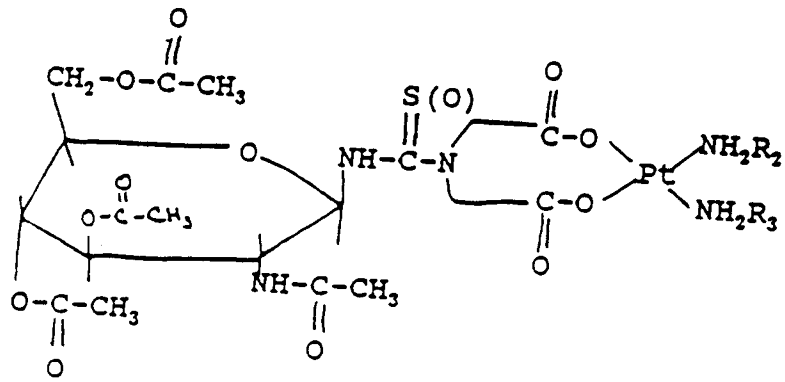

et $R_2$ et $R_3$ sont tels que définis ci-dessus.

**16.** Composé de formule :

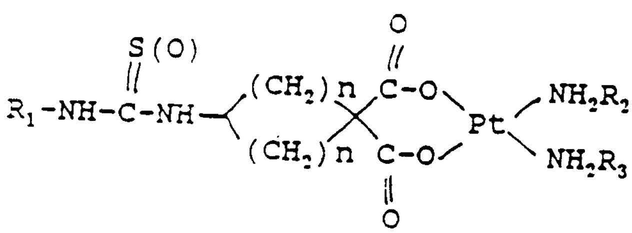

dans laquelle n est égal à 1 ou 2; $R_1$ est un mono- ou un di-saccharide, ou un dérivé de ceux-ci; chacun des radicaux $R_2$ et $R_3$ est choisi indépendamment dans le groupe constitué par un hydrogène ou un groupe alkyle en $C_1$-$C_4$, ou $R_2$ et $R_3$ sont liés ensemble à des atomes de carbone adjacents sur une structure cyclique à cinq ou six chaînons

ou sel pharmaceutiquement acceptable de ce composé.

**17.** Composé selon la revendication 16, dans lequel le composé a pour formule :

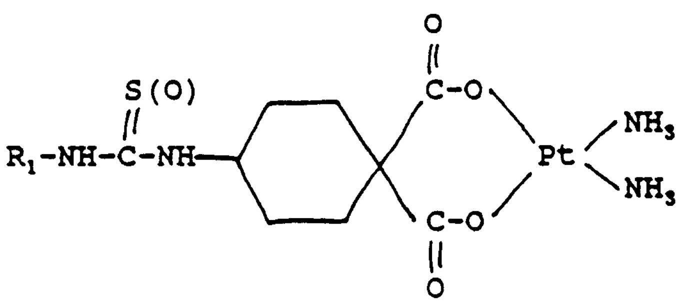

et $R_1$ est choisi dans le groupe comprenant le glucose, le mannose, le galactose, la glucosamine, la galactosamine et des dérivés de ceux-ci.

**18.** Composé selon la revendication 16, dans lequel le composé a pour formule :

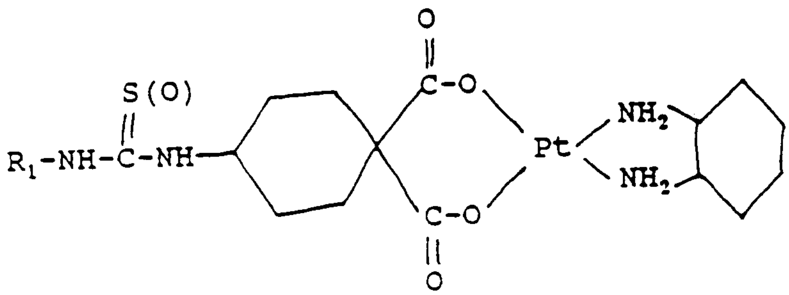

et $R_1$ est choisi dans le groupe comprenant le glucose, le mannose, le galactose, la glucosamine, la galactosamine et des dérivés de ceux-ci.

**19.** Composé de formule :

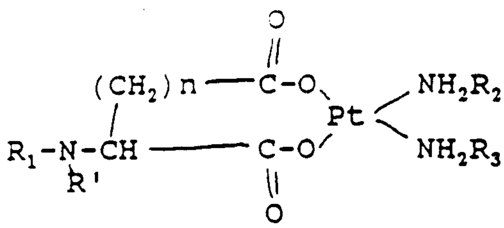

dans laquelle n est égal à 0 ou 1, $R_1$ est choisi dans le groupe constitué par un hydrogène, un mono- ou un di-saccharide, ou un dérivé de ceux-ci, lié à l'atome d'azote par un groupement amide -NHCO-, un groupement thioamide -NHCS- ou un groupement carbonyle -CO-, R' est choisi dans le groupe constitué par un hydrogène, un groupe alkyle en $C_1$-$C_4$, et $R_2$ et $R_3$ sont choisis dans le groupe constitué par un hydrogène, un groupe alkyle en $C_1$-$C_4$, ou $R_2$ et $R_3$ sont liés ensemble à des atomes de carbone adjacents sur une structure cyclique à quatre, cinq ou six chaînons, ou $R_2$ et $R_3$ forment ensemble un noyau condensé ou bicyclique avec les atomes de carbone adjacents; à condition que R' et $R_1$ ne soient pas tous deux des atomes d'hydrogène lorsque $n = 0$;

ou sel pharmaceutiquement acceptable de ce composé.

**20.** Composé selon la revendication 19, dans lequel $R_1$ est un mono- ou un di-saccharide, ou un dérivé de ceux-ci, choisi dans le groupe comprenant le glucose, le galactose, le mannose, la glucosamine et la galactosamine, et des dérivés de ceux-ci.

**21.** Composé selon la revendication 19, dans lequel $R_2$ et $R_3$ sont des atomes d'hydrogène.

**22.** Composé selon la revendication 19, dans lequel $R_2$ et $R_3$ ensemble sont liés à des atomes de carbone adjacents sur une structure cyclique à quatre, cinq ou six chaînons.

**23.** Composé selon la revendication 19, dans lequel $R_2$ et $R_3$ forment ensemble un noyau condensé ou bicyclique avec les atomes de carbone adjacents.

**24.** Composé selon la revendication 19, dans lequel $R_2$ et $R_3$ forment ensemble un groupe de formule suivante :

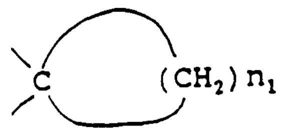

dans laquelle $n_1$ est choisi parmi 1, 2, 3, 4, 5 ou 6.

**25.** Composé selon la revendication 19, dans lequel $R_2$ et $R_3$ forment ensemble un groupe de formule suivante :

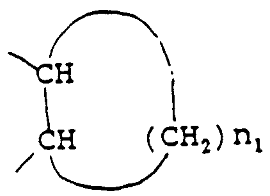

dans laquelle $n_1$ est choisi parmi 1, 2, 3, 4, 5 ou 6.

**26.** Composé selon la revendication 19, dans lequel ledit composé a pour formule :

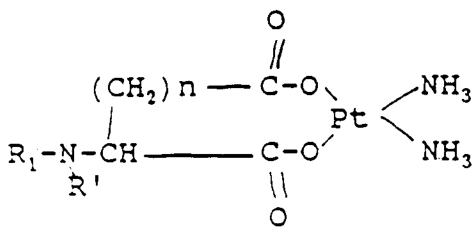

et $R_1$ et R' sont tels que définis dans la revendication 19.

**27.** Composé selon la revendication 19, dans lequel ledit composé a pour formule :

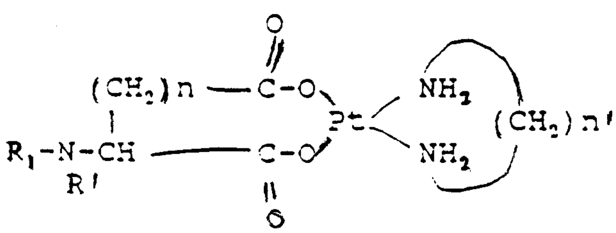

et $R_1$ et R' sont tels que définis dans la revendication 19 et n' est égal à 1, 2 ou 3.

**28.** Composé selon la revendication 19, dans lequel ledit composé est le (L-aspartato-O,O')-(1,2-cyclohexanediammine-N,N')-platine(II).

**29.** Composé selon la revendication 19, dans lequel ledit composé est le diammine-2-[[[[3,4,6-tri-O-acétyl-2-(N-acétylamino)-2-désoxy-α-D-glucopyranosyl]amino]thioxométhyl]amino]butanedioato-O,O']-platine (II).

**30.** Composé selon la revendication 19, dans lequel ledit composé est le 2-[[[[3,4,6-tri-O-acétyl-2-(N-acétylamino)-2-désoxy-α-D-glucopyranosyl]amino]thioxométhyl]amino]butanedioato-O,O']-(1,2-cyclohexanediammine-N,N')-platine (II).

**31.** Composition pharmaceutique comprenant un composé selon l'une quelconque des revendications 1 à 30 comme ingrédient actif.